# EUROPEAN PATENT APPLICATION

(11) **EP 4 603 585 A1**
(43) Date of publication of application: **20.08.2025**
(21) Application number: 23876548.1
(22) Date of filing: 27.09.2023
(51) Int. Cl.: C12N 15/113, A61K 31/7088, A61K 48/00

(54) **SNRNA NUCLEIC ACID MOLECULE AND APPLICATION THEREOF**

(30) Priority: 11.10.2022 CN 202211243389
(71) Applicant: Guangzhou Reforgene Medicine Co., Ltd., Guangzhou, Guangdong 510535 (CN)
(72) Inventor: LIANG, Junbin, Guangzhou, Guangdong 510535 (CN); OU, Jiayu, Guangzhou, Guangdong 510535 (CN); XU, Hui, Guangzhou, Guangdong 510535 (CN)
(74) Representative: Murgitroyd & Company
(86) International application number: PCT/CN2023/122201
(87) International publication number: WO 2024/078345

(57) **Abstract**

Provided are an snRNA nucleic acid molecule and an application thereof. The snRNA nucleic acid molecule comprises: a recognition domain, a stem-loop sequence, and an Sm sequence; wherein the number of recognition domains is at least two; wherein each recognition domain from the 5' end to the 3' end is reverse complementary to target sequence segments from the 3' end to the 5' end of pre-mRNA; the pre-mRNA being pre-mRNA corresponding to the USH2A gene. The snRNA nucleic acid molecule can efficiently induce USH2A exon 13 skipping, with a higher single-skip reading frequency. Especially regarding pathogenic or non-pathogenic USH2A exon 13, the present invention can safely and effectively read USH2A exon 13 single skips at low doses, and has important clinical value in the prevention and/or treatment of eye diseases and ear diseases related to abnormal expression of the Usherin protein.

## Description

### TECHNICAL FIELD

The present disclosure belongs to the field of biotechnology, and specifically relates to an snRNA nucleic acid molecule and application thereof.

### BACKGROUND

Usher syndrome belongs to a class of genetic diseases, also known as deafness-retinitis pigmentosa syndrome, which is characterized by varying degrees of congenital sensorineural hearing loss and retinitis pigmentosa (RP)-induced progressive vision loss. Clinically, Usher syndrome can be divided into three types: (1) Type I Usher syndrome: profound congenital sensorineural hearing loss, loss of vestibular response, and retinitis pigmentosa before puberty, leading to gradual blindness, and associated genes include MYO7A, CDH23, USH1C, PCHD15, *etc*.; (2) Type II Usher syndrome: moderate to severe congenital sensorineural hearing loss, normal vestibular response, and retinitis pigmentosa during puberty, leading to gradual blindness, and associated genes include USH2A, GPR98, WHRN, *etc*.; (3) Type III Usher syndrome: progressive sensorineural hearing loss, normal vestibular response, and retinitis pigmentosa at the end of puberty, leading to gradual blindness, and associated genes include CLRN1, *etc.*

Type II Usher syndrome accounts for more than 50% of Usher syndrome, and mutations in the USH2A gene are the most common cause of Type II Usher syndrome, covering more than 50% of patients with Usher syndrome. Meanwhile, mutations in the USH2A gene are also one of the important causes of nonsyndromic retinitis pigmentosa (NSRP).

The USH2A gene, which is localized at 1q41 and spans more than 800 kb in a genome, encodes a large transmembrane protein, Usherin, which is anchored to the plasma membrane of retinal photoreceptor cells and inner ear hair cells, and thus is an essential component for cilia development and maintenance. In the retina, Usherin is a crucial part of the USH2 complex and is regarded to play a role in stabilizing the outer segments of photoreceptors. USH2A has two isoforms, with the predominant isoform in retinal cells containing 72 exons, each having a coding region of approximately 15.6 kb in length. The extracellular portion of a Usherin protein contains numerous repetitive domains, including 10 Laminin EGF-like (LE) domains and 35 Fibronectin type 3 (FN3) domains. Exon 13 of human USH2A is 642 bp in length and encodes amino acids 723 to 936 of Usherin, constituting 4 of the 10 LE domains in the Usherin protein.

Mutations in exon 13, exon 50, and intron 40 of the USH2A gene cause Usher syndrome. To date, more than 1,000 pathogenic mutations have been identified throughout the USH2A gene, with exon 13 being the most frequently mutated exon in the USH2A gene, accounting for approximately 35% of the mutations. Mutations in exon 13 of the USH2A gene include c.2802T>G (p.Cys934Trp), c.2299delG (p.Glu767Serfs*21, the most frequent mutation in European and American patients), c.2276G>T (p.cys759phe, the most common mutation site in nonsyndromic RP), c.2522C>A (p.S841Y), c.2242C>T (p.Gln748X), c.2541C>A(C847X), c.2761delC (Leu921fs), c.2776C>T (p.R926C), c.2209C>T, c.2310delA, c.2391_2392deITG, c.2431A>T, c.2431_2432delAA, c.2440C>T, c.2525dup, c.2610C>A, c.2755C>T, c.2176T>C, c.2236C>G, c.2296T>C, c.2332G>T, and c.2339G>T (PMID: 31904091).

The coding region of USH2A is approximately 15.6 kb in length, and such a large coding sequence is hardly packaged by conventional gene therapy delivery methods (e.g., recombinant lentivirus and recombinant adeno-associated virus), making it difficult to directly deliver USH2A for treatment. Exon 12 of mouse USH2A is homologous to exon 13 of human USH2A, both being 642 bp in length, and removal of the exon does not result in subsequent frameshift mutations. Studies have shown that after knocking out exon 12 of mouse USH2A, Usherin is still able to be correctly localized and perform normal functions. By analogy, for exon 13 of human USH2A containing pathogenic mutations, a series of means can be employed to induce exon skipping for treatment.

In the prior art, genomic DNA is edited by the CRISPR/Cas system to delete exon 13 directly or disrupt RNA splicing-related sites. There are risks associated with the use of fragment deletions, such as chromosomal rearrangements, viral integration, reverse reintegration, and a high off-target probability of long-term expression of the CAS system or double cleavage induced by two gRNAs based on a relatively large genomic background.

In the prior art, the exon skipping can also be promoted by using a single-base editor to modify the key bases at the splicing-related sites described above. However, the existing single-base editors cannot be packaged into a single AAV vector and may not have suitable gRNAs near the splicing-related sites, limited by the PAM, editing window, and type of base conversion.

In the prior art, targeted interference with pre-mRNA splicing using antisense oligonucleotides (AONs) is shown to have high efficiency of exon skipping. However, AONs promote the co-skipping of human exons 12 and 13 while promoting the skipping of human exon 13. Some AON treatments even result in double skipping without single skipping. As human exon 12 is 196 bp in full-length, not an integer multiple of 3, its deletion results in a frameshift mutation and inactivation of the USH2A protein.

US10131910B2 and US10745699B2 from Catholic University Foundation disclose antisense oligonucleotides for the treatment of Type II Usher syndrome. These patents utilize antisense oligonucleotides (AONs) targeting exon 13 (intron 12) of human USH2A, PE40, and exon 50 to induce exon skipping and intron 12 retention.

Although these patents utilize AON4a targeting intron 12 to effectively retain exon 12, the use of multiple AONs increases the risk of off-target effects. Meanwhile, the high demand for AON4a results in an even higher overall AON dosage, which poses a risk of cytotoxicity.

RPOQR (CN109804069A) discloses antisense oligonucleotides for the treatment of eye diseases. The patent identified three sites, Ex13-1, Ex13-2, and Ex13-3, that appear to produce stronger signals for single (exon 13) skipping based on US10131910B2 and US10745699B2.

However, AONs promote the co-skipping of human exons 12 and 13 while promoting the skipping of human exon 13. Some AON treatments even result in mostly double skipping or the presence of non-specific bands due to aberrant/complete skipping of exon 13 (potentially due to single skipping of exon 12). As human exon 12 is 196 bp in full-length, not an integer multiple of 3, its deletion results in a frameshift mutation and inactivation of the USH2A protein after double skipping. The effects of AONs are not long-lasting, requiring frequent administration, and the efficiency of induced splicing skipping is not high. Moreover, the dosage of AONs is very high.

There are small nuclear RNAs (snRNAs) in cells, which are major components of RNA spliceosomes during post-transcriptional processing in eukaryotes and are involved in the processing of mRNA precursors by binding to snRNP proteins. snRNAs, approximately 100 to 215 nucleotides in length in mammals, are divided into 7 types, numbered U1 to U7 due to its abundance of U. However, U7 snRNP is not involved in splicing but is instead a key factor in the unique 3' end processing of replication-dependent histone (RDH) pre-mRNA. The modified U7 snRNA is prepared by replacing the non-canonical Sm binding site of U7 snRNA with the consensus sequence derived from the major spliceosomal U snRNPs and changing the histone binding sequence at the 5' region of U7 snRNA into the complementary sequence of the gene to be modified, which can induce exon splicing skipping by targeting exons.

However, since the development of modified U7 snRNAs in 1998, the related research and applications have not been extensive and are limited to only a few targets, restricted by the number of vectors for U7 snRNP administration. For example, the delivery of modified U7 snRNAs with viruses such as AAVs require very high viral doses, which may pose toxicity risks or trigger immune responses, limiting the application of viral delivery of U7 snRNAs. Other long-term delivery methods involving gene integration pose genomic safety risks, while transient delivery methods offer short-lived effects.

### SUMMARY

The technical problem to be solved by the present disclosure is to overcome the defect of low efficiency of inducing splicing skipping of exon 13 of USH2A in the prior art by providing an snRNA nucleic acid molecule and use thereof. The present disclosure promotes the efficiency of single exon skipping by targeted interference with pre-mRNA splicing of exon 13 of USH2A using snRNA, which significantly improves the efficiency while ensuring safety.

The present disclosure solves the above technical problem through the following technical solutions.

The first aspect of the present disclosure provides an snRNA nucleic acid molecule, comprising: a recognition domain, a stem-loop sequence, and an Sm sequence; wherein the number of the recognition domains is at least two;

wherein each of the recognition domains from the 5' end to the 3' end is reverse complementary to a target sequence fragment from the 3' end to the 5' end of a pre-mRNA; the pre-mRNA is a pre-mRNA corresponding to the USH2A gene.

In some embodiments of the present disclosure, each of the recognition domains from the 5' end to the 3' end is sequentially reverse complementary to the target sequence fragment from the 3' end to the 5' end of the pre-mRNA, meaning that the recognition domains from the 5' end to the 3' end of the snRNA nucleic acid molecule are ordered from the 3' end to the 5' end based on the positions of the target sites corresponding to the recognition domains in the USH2A pre-mRNA.

In some embodiments of the present disclosure, each of the recognition domains from the 5' end to the 3' end is non-sequentially reverse complementary to the target sequence fragment from the 3' end to the 5' end of the pre-mRNA, meaning that the recognition domains from the 5' end to the 3' end of the snRNA nucleic acid molecule are not ordered from the 3' end to the 5' end based on the positions of the target sites corresponding to the recognition domains in the USH2A pre-mRNA.

In some embodiments of the present disclosure, the recognition domain has a length of at least 16 bp.

In some other embodiments of the present disclosure, the recognition domain has a length of 18 to 40 bp.

In some other embodiments of the present disclosure, the recognition domain has a length of 20 to 27 bp.

In some other embodiments of the present disclosure, the recognition domain has a length of 22 to 27 bp.

In some embodiments of the present disclosure, the number of the recognition domains is two; preferably, the two recognition domains are adjacent to each other.

In some embodiments of the present disclosure, the number of the stem-loop sequences may be 1 to 2.

In some specific embodiments of the present disclosure, the snRNA nucleic acid molecule comprises, sequentially from the 5' end to the 3' end: two adjacent recognition domains, an Sm sequence, and a stem-loop sequence.

In some embodiments of the present disclosure, the pre-mRNA is all or part of the pre-mRNA corresponding to intron 12 to intron 13 of the USH2A gene.

In some preferred embodiments of the present disclosure, the pre-mRNA is all or part of the pre-mRNA corresponding to exon 13 of the USH2A gene.

In some embodiments of the present disclosure, the genomic location of the pre-mRNA is Chr1: 216246563-216247246; the target sequence fragment is selected from the nucleotide sequence as shown in SEQ ID NO: 1 and a variant sequence thereof.

In some other embodiments of the present disclosure, the genomic location of the pre-mRNA is Chr1: 216246563-216246753; the target sequence fragment is selected from the nucleotide sequence as shown in SEQ ID NO: 3 and a variant sequence thereof.

In some preferred embodiments of the present disclosure, the genomic location of the pre-mRNA is Chr1: 216246563-216246649; the target sequence fragment is selected from the nucleotide sequence as shown in SEQ ID NO: 4 and a variant sequence thereof.

In some specific embodiments of the present disclosure, the genomic location of the pre-mRNA is Chr1: 216246563-216246626; the target sequence fragment is selected from the nucleotide sequence as shown in SEQ ID NO: 9 and a variant sequence thereof.

In some specific embodiments of the present disclosure, the genomic location of the pre-mRNA is Chr1: 216246616-216246649; the target sequence fragment is selected from the nucleotide sequence as shown in SEQ ID NO: 34 and a variant sequence thereof.

In some specific embodiments of the present disclosure, the genomic location of the pre-mRNA is Chr1: 216247130-216247246; the target sequence fragment is selected from the nucleotide sequence as shown in SEQ ID NO: 2 and a variant sequence thereof.

In some specific embodiments of the present disclosure, the genomic location of the pre-mRNA is Chr1: 216247142-216247185; the target sequence fragment is selected from the nucleotide sequence as shown in SEQ ID NO: 32 and a variant sequence thereof.

In some specific embodiments of the present disclosure, the genomic location of the pre-mRNA is Chr1: 216247130-216247161; the target sequence fragment is selected from the nucleotide sequence as shown in SEQ ID NO: 33 and a variant sequence thereof.

In some specific embodiments of the present disclosure, the genomic location of the pre-mRNA is Chr1: 216247210-216247246; the target sequence fragment is selected from the nucleotide sequence as shown in SEQ ID NO: 36 and a variant sequence thereof.

In some specific embodiments of the present disclosure, the genomic location of the pre-mRNA is Chr1: 216247204-216247232; the target sequence fragment is selected from the nucleotide sequence as shown in SEQ ID NO: 37 and a variant sequence thereof.

In some specific embodiments of the present disclosure, the genomic location of the pre-mRNA is Chr1: 216247187-216247220; the target sequence fragment is selected from the nucleotide sequence as shown in SEQ ID NO: 38 and a variant sequence thereof.

In some specific embodiments of the present disclosure, the genomic location of the pre-mRNA is Chr1: 216247169-216247202; the target sequence fragment is selected from the nucleotide sequence as shown in SEQ ID NO: 39 and a variant sequence thereof.

In the present disclosure, the two recognition domains are, from the 5' end to the 3' end, a first recognition domain and a second recognition domain, respectively; the first recognition domain and the second recognition domain may be RNA sequences recognizing different target sites or RNA sequences recognizing the same target site.

In some specific embodiments of the present disclosure, in RNA sequences recognizing different target sites, the reverse complementary RNA sequence binding to the 3' end of the USH2A pre-mRNA serves as the second recognition domain of the snRNA, and the reverse complementary RNA binding to the 5' end of the USH2A pre-mRNA serves as the first recognition domain of the snRNA.

In some embodiments of the present disclosure, the target sequence fragment reverse complementary to the first recognition domain or the second recognition domain is selected from the nucleotide sequence as shown in SEQ ID NO: 34 and a variant sequence thereof and the nucleotide sequence as shown in SEQ ID NO: 9 and a variant sequence thereof; correspondingly, the target sequence fragment reverse complementary to the second recognition domain or the first recognition domain is selected from the nucleotide sequence as shown in SEQ ID NO: 32 and a variant sequence thereof, the nucleotide sequence as shown in SEQ ID NO: 33 and a variant sequence thereof, the nucleotide sequence as shown in SEQ ID NO: 36 and a variant sequence thereof, the nucleotide sequence as shown in SEQ ID NO: 37 and a variant sequence thereof, the nucleotide sequence as shown in SEQ ID NO: 38 and a variant sequence thereof, and the nucleotide sequence as shown in SEQ ID NO: 39 and a variant sequence thereof.

In some embodiments of the present disclosure, the target sequence fragment reverse complementary to the first recognition domain is selected from the nucleotide sequence as shown in SEQ ID NO: 34 and a variant sequence thereof and the nucleotide sequence as shown in SEQ ID NO: 9 and a variant sequence thereof; the target sequence fragment reverse complementary to the second recognition domain is selected from the nucleotide sequence as shown in SEQ ID NO: 32 and a variant sequence thereof, the nucleotide sequence as shown in SEQ ID NO: 33 and a variant sequence thereof, the nucleotide sequence as shown in SEQ ID NO: 36 and a variant sequence thereof, the nucleotide sequence as shown in SEQ ID NO: 37 and a variant sequence thereof, the nucleotide sequence as shown in SEQ ID NO: 38 and a variant sequence thereof, and the nucleotide sequence as shown in SEQ ID NO: 39 and a variant sequence thereof.

In the present disclosure, the variant sequence is a sequence obtained by substitution, addition, or deletion of one or more nucleotides based on the start sequence.

In some specific embodiments of the present disclosure, the nucleotide sequence of the first recognition domain is as shown in any one of SEQ ID NOs: 12 to 22 and 59 to 61, and the nucleotide sequence of the second recognition domain is as shown in any one of SEQ ID NOs: 40 to 58.

In some specific embodiments of the present disclosure, the nucleotide sequence of the first recognition domain is as shown in SEQ ID NO: 12, 13, 15, or 17, and the nucleotide sequence of the second recognition domain is as shown in SEQ ID NO: 48, 54, 56, or 58.

In some specific embodiments of the present disclosure, the nucleotide sequence of the first recognition domain is as shown in SEQ ID NO: 12, 13, or 17, and the nucleotide sequence of the second recognition domain is as shown in SEQ ID NO: 48, 54, or 58.

In some embodiments of the present disclosure, the nucleotide sequence of the first recognition domain is as shown in SEQ ID NO: 12, and the nucleotide sequence of the second recognition domain is as shown in SEQ ID NO: 48.

In some other embodiments of the present disclosure, the nucleotide sequence of the first recognition domain is as shown in SEQ ID NO: 12, and the nucleotide sequence of the second recognition domain is as shown in SEQ ID NO: 54.

In some other embodiments of the present disclosure, the nucleotide sequence of the first recognition domain is as shown in SEQ ID NO: 12, and the nucleotide sequence of the second recognition domain is as shown in SEQ ID NO: 58.

In some other embodiments of the present disclosure, the nucleotide sequence of the first recognition domain is as shown in SEQ ID NO: 13, and the nucleotide sequence of the second recognition domain is as shown in SEQ ID NO: 48.

In some other embodiments of the present disclosure, the nucleotide sequence of the first recognition domain is as shown in SEQ ID NO: 13, and the nucleotide sequence of the second recognition domain is as shown in SEQ ID NO: 54.

In some other embodiments of the present disclosure, the nucleotide sequence of the first recognition domain is as shown in SEQ ID NO: 13, and the nucleotide sequence of the second recognition domain is as shown in SEQ ID NO: 58.

In some other embodiments of the present disclosure, the nucleotide sequence of the first recognition domain is as shown in SEQ ID NO: 17, and the nucleotide sequence of the second recognition domain is as shown in SEQ ID NO: 48.

In some other embodiments of the present disclosure, the nucleotide sequence of the first recognition domain is as shown in SEQ ID NO: 17, and the nucleotide sequence of the second recognition domain is as shown in SEQ ID NO: 54.

In some other embodiments of the present disclosure, the nucleotide sequence of the first recognition domain is as shown in SEQ ID NO: 17, and the nucleotide sequence of the second recognition domain is as shown in SEQ ID NO: 58.

In some other embodiments of the present disclosure, the nucleotide sequence of the first recognition domain is as shown in SEQ ID NO: 16, and the nucleotide sequence of the second recognition domain is as shown in SEQ ID NO: 42.

In some other embodiments of the present disclosure, the nucleotide sequence of the first recognition domain is as shown in SEQ ID NO: 18, and the nucleotide sequence of the second recognition domain is as shown in SEQ ID NO: 43.

In some other embodiments of the present disclosure, the nucleotide sequence of the first recognition domain is as shown in SEQ ID NO: 14, and the nucleotide sequence of the second recognition domain is as shown in SEQ ID NO: 55.

In the present disclosure, the variant sequence is the presence of a substitution, addition, or deletion, preferably a substitution, of one or more nucleotides in the nucleotide sequence.

In the present disclosure, the mutation is selected from a natural pathogenic mutation and a natural non-pathogenic mutation. The natural pathogenic mutation is one or more selected from c.2242C>T, c.2276G>T, c.2299delG, c.2522C>A, c.2541C>A, c.2761delC, c.2776C>T, c.2802T>G, c.2209C>T, c.2310delA, c.2391_2392deITG, c.2431A>T, c.2431_2432delAA, c.2440C>T, c.2525dup, c.2610C>A, c.2755C>T, c.2176T>C, c.2236C>G, c.2296T>C, and c.2332G>T.

In some specific embodiments of the present disclosure, the natural pathogenic mutation is one or more selected from c.2802T>G, c.2299delG, and c.2276G>T; for example, the nucleotide mutation is c.2802T>G.

In some embodiments of the present disclosure, the Sm sequence is a consensus sequence, and the stem-loop sequence may be a stem-loop sequence of U1, U2, U3, U4, U5, U6, or U7.

In some specific embodiments of the present disclosure, the stem-loop sequence is a stem-loop sequence of U7.

In some specific embodiments of the present disclosure, the stem-loop sequence is a stem-loop sequence of U1.

In some specific embodiments of the present disclosure, the Sm sequence is as shown in SEQ ID NO: 6.

In some specific embodiments of the present disclosure, the stem-loop sequence is as shown in SEQ ID NO: 7.

In some embodiments of the present disclosure, the snRNA nucleic acid molecule comprises a modified nucleotide or an analog monomer thereof.

In the present disclosure, the modification is selected from a 2'-O-alkyl modification, a 2'-O-methoxy modification, and a 2'-O-methoxyethyl modification.

In some specific embodiments of the present disclosure, the 2'-O-alkyl modification is a 2'-O-methyl modification.

In some embodiments of the present disclosure, the nucleotide analog monomer is selected from a 6'-modified bicyclic nucleoside, a 5'-modified bicyclic nucleoside, a 6'-disubstituted bicyclic nucleoside, a tetrahydropyran nucleoside analog, and a 2'-deoxy-2'-fluoro-β-D-arabinonucleotide.

In some embodiments of the present disclosure, the nucleotides of the snRNA nucleic acid molecule are linked by a chemical bond, and the chemical bond is selected from a phosphate bond, a methylene bond, an amide bond, a methylphosphonate bond, and a 3'-thioacetal bond.

In some embodiments of the present disclosure, the phosphate bond is selected from a phosphorothioate bond, a dithiophosphate bond, an alkylphosphonate bond, a phosphoroamidate bond, a boranophosphate bond, and a chiral linkage phosphorus.

In some specific embodiments of the present disclosure, the phosphate bond is a phosphorothioate bond.

In some embodiments of the present disclosure, the snRNA nucleic acid molecule comprises a modified nucleotide or an analog monomer thereof at positions 1 to 80 from the 5' end and/or the 3' end.

In some other embodiments of the present disclosure, the snRNA nucleic acid molecule comprises a modified nucleotide or an analog monomer thereof at positions 3 to 40 from the 5' end and/or the 3' end.

In some other embodiments of the present disclosure, the snRNA nucleic acid molecule comprises a modified nucleotide or an analog monomer thereof at positions 6 to 10 from the 5' end and/or the 3' end.

In some other embodiments of the present disclosure, the snRNA nucleic acid molecule comprises a modified nucleotide or an analog monomer thereof at positions 20 to 27 from the 5' end and/or the 3' end.

In some specific embodiments of the present disclosure, the snRNA nucleic acid molecule comprises at least one phosphate bond from the 5' end or the 3' end.

In some embodiments of the present disclosure, the snRNA nucleic acid molecule comprises one or more of the modifications as described above.

In the present disclosure, when the snRNA nucleic acid molecule is chemically synthesized, all nucleotides are linked to each other via a phosphorothioate bond and subjected to 2'-O-methoxy modification. In some embodiments, only three nucleotides at both ends of the snRNA are linked via a phosphorothioate bond and subjected to 2'-O-methoxy modification. In some embodiments of chemically synthesized and modified U7 snRNA, there may be 0 to 5 mismatched nucleotides, preferably 0 to 1 mismatched nucleotide in the reverse complementary pairing between the recognition domain and the target site. In some embodiments, 3 to 40 bases at both ends of the chemically synthesized snRNA are modified and linked via a phosphate bond.

In some specific embodiments of the present disclosure, the snRNA nucleic acid molecule comprises 1 to 3 phosphate bonds from the 5' end.

In some specific embodiments of the present disclosure, the snRNA nucleic acid molecule comprises 1 to 3 phosphate bonds from the 3' end.

In some embodiments of the present disclosure, the snRNA nucleic acid molecule further comprises a unidirectional extension sequence or a bidirectional extension sequence on the nucleotides at the 5' end and/or the 3' end of the recognition domain.

The unidirectional extension sequence is an RNA sequence added to the 5' end or the 3' end of the target sequence of the snRNA nucleic acid molecule; the bidirectional extension sequence is an RNA sequence added to both the 5' end and the 3' end of the target sequence of the snRNA nucleic acid molecule, respectively.

In some embodiments of the present disclosure, the snRNA nucleic acid molecule further comprises a free tail sequence, wherein the tail sequence comprises a motif of a splicing regulatory protein and can bind to the splicing regulatory protein.

In some specific embodiments of the present disclosure, the splicing regulatory protein is selected from hnRNP A1 (Heterogeneous Nuclear Ribonucleoprotein A1), SRSF1 (Serine and Arginine Rich Splicing Factor 1), RBM4 (RNA Binding Motif Protein 4), DAZAP1 (DAZ Associated Protein 1), and SR (Serine and Arginine-Rich Protein).

For example, when the splicing regulatory protein is hnRNP A1, the tail sequence is as shown in SEQ ID NO: 35.

A second aspect of the present disclosure provides a combination of snRNA nucleic acid molecules, comprising one or more snRNA nucleic acid molecules according to the first aspect.

In some embodiments of the present disclosure, at least two recognition domains are located on the same or different snRNA nucleic acid molecules.

A third aspect of the present disclosure provides a DNA molecule encoding the snRNA nucleic acid molecule according to the first aspect or the combination according to the first aspect.

A fourth aspect of the present disclosure provides a gene expression cassette, comprising a promoter and the DNA molecule according to the third aspect.

In some embodiments of the present disclosure, the promoter is a U7 promoter.

In some specific embodiments of the present disclosure, the promoter is a murine U7 promoter.

In some embodiments of the present disclosure, the gene expression cassette comprises a tailing sequence at the 3' end, wherein the tailing sequence is involved in the processing of the snRNA.

In the present disclosure, the tailing sequence has a length of 28 to 131 bp, for example, 106 bp.

In some embodiments of the present disclosure, the tailing sequence is a gene sequence following the 3' end of the U7 snRNA gene, for example, as shown in SEQ ID NO: 8.

In some embodiments of the present disclosure, the gene expression cassette comprises a recognition domain and a scaffold sequence; the scaffold sequence is as shown in SEQ ID NO: 62.

A fifth aspect of the present disclosure provides a recombinant expression vector, comprising the snRNA nucleic acid molecule according to the first aspect, the combination according to the second aspect, or the gene expression cassette according to the third aspect.

In some embodiments of the present disclosure, the recombinant expression vector is an expression vector selected from a plasmid, a phage, a minicircle DNA, a linear DNA, and a virus.

In some specific embodiments of the present disclosure, the expression vector is a lentivirus or an adeno-associated virus.

In the present disclosure, the capsid protein of the adeno-associated virus is a naturally derived capsid protein or a mutant thereof, and the plasmid of the adeno-associated virus is a single-stranded AAV (ssAAV) or a self-complementary AAV (scAAV).

In the present disclosure, the naturally derived AAV capsid protein may be derived from animals or plants. The AAV capsid protein derived from animals may be derived from humans (*e.g*., AAV1, AAV2, AAV3, AAV4, AAV5, AAV6, AAV7, AAV8, and AAV9), non-human primates (*e.g*., AAVrh.8, AAVrh.10, and AAVrh.43), vertebrates such as mice and pigs, or insects. Preferably, an AAV serotype with tropism for retinal tissue, such as AAV1, AAV2, AAV4, AAV5, AAV7, AAV8, AAV9, AAVrh10, or AAV2.7m8. In the AAV plasmid system of the present disclosure, for example, the AAV ITR serotype should be consistent with the Rep gene serotype, but may not be consistent with the Cap gene serotype. The AAV may be a single-stranded AAV (ssAAV) or a self-complementary AAV (scAAV).

In some specific embodiments of the present disclosure, the naturally-derived capsid protein is selected from AAV1, AAV2, AAV3, AAV4, AAV5, AAV6, AAV7, AAV8, AAV9, AAVrh.8, AAVrh. 10, and AAVrh.43;
the mutant is selected from AAV2.5, AAV2i8, AAV-TT, AAV9.HR, and CAM130.

A sixth aspect of the present disclosure provides a virus particle, comprising a capsid protein and a nucleic acid, wherein the nucleic acid comprises the snRNAnucleic acid molecule according to the first aspect, the combination according to the second aspect, or the DNA molecule according to the third aspect.

In some embodiments of the present disclosure, the capsid protein is a capsid protein derived from an adeno-associated virus.

In some embodiments of the present disclosure, the capsid protein derived from the adeno-associated virus is defined in the fifth aspect.

A seventh aspect of the present disclosure provides a pharmaceutical composition, comprising the snRNA nucleic acid molecule according to the first aspect, the combination according to the second aspect, the DNA molecule according to the third aspect, the gene expression cassette according to the fourth aspect, the recombinant expression vector according to the fifth aspect, or the virus particle according to the sixth aspect.

In some embodiments of the present disclosure, the pharmaceutical composition further comprises a pharmaceutically acceptable carrier.

An eighth aspect of the present disclosure provides a method for inducing the production of an Usherin protein lacking exon 13, comprising introducing into a host cell the snRNA nucleic acid molecule according to the first aspect, the combination according to the second aspect, the DNA molecule according to the third aspect, the gene expression cassette according to the fourth aspect, the recombinant expression vector according to the fifth aspect, the virus particle according to the sixth aspect, or the pharmaceutical composition according to the seventh aspect, thereby causing splicing skipping of exon 13.

In some embodiments of the present disclosure, the host cell is selected from retinal tissue cells, inner ear cells, cells with differentiation potential into retinal tissue cells and/or inner ear cells, and cells capable of performing functions corresponding to retinal tissue cells and/or inner ear cells.

In some specific embodiments of the present disclosure, the retinal tissue cells are retinal photoreceptor cells, and the inner ear cells are inner ear hair cells.

In the present disclosure, the stem cells are selected from induced pluripotent stem cells and embryonic stem cells.

In the present disclosure, the cells with differentiation potential are selected from induced pluripotent stem cells, embryonic stem cells, neural precursor cells, retinal progenitor cells, retinal precursor cells, and mesenchymal stromal cells.

A ninth aspect of the present disclosure provides a method for inhibiting the expression and/or function of exon 13 of USH2A pre-mRNA, comprising administering the snRNA nucleic acid molecule according to the first aspect, the combination according to the second aspect, the DNA molecule according to the third aspect, the gene expression cassette according to the fourth aspect, the recombinant expression vector according to the fifth aspect, the virus particle according to the sixth aspect, or the pharmaceutical composition according to the seventh aspect.

A tenth aspect of the present disclosure provides a method for inducing splicing skipping of exon 13 of USH2A pre-mRNA, comprising administering the snRNA nucleic acid molecule according to the first aspect, the combination according to the second aspect, the DNA molecule according to the third aspect, the gene expression cassette according to the fourth aspect, the recombinant expression vector according to the fifth aspect, the virus particle according to the sixth aspect, or the pharmaceutical composition according to the seventh aspect.

An eleventh aspect of the present disclosure provides a method for reducing the abnormal expression of an Usherin protein, comprising introducing into a host cell the snRNA nucleic acid molecule according to the first aspect, the combination according to the second aspect, the DNA molecule according to the third aspect, the gene expression cassette according to the fourth aspect, the recombinant expression vector according to the fifth aspect, the virus particle according to the sixth aspect, or the pharmaceutical composition according to the seventh aspect.

In some embodiments of the present disclosure, the host cell is as defined in the eighth aspect.

A twelfth aspect of the present disclosure provides a method for preparing the snRNA nucleic acid molecule according to the first aspect or the combination according to the second aspect, comprising the step of biosynthesis or chemical synthesis of the snRNA nucleic acid molecule according to the first aspect or the combination according to the second aspect.

A thirteenth aspect of the present disclosure provides use of the snRNA nucleic acid molecule according to the first aspect, the combination according to the second aspect, the DNA molecule according to the third aspect, the gene expression cassette according to the fourth aspect, the recombinant expression vector according to the fifth aspect, the virus particle according to the sixth aspect, or the pharmaceutical composition according to the seventh aspect in the manufacture of a medicament for treating a disease associated with a mutation in exon 13 of USH2A.

In some embodiments of the present disclosure, the mutation in exon 13 of USH2Ais a pathogenic mutation or a non-pathogenic mutation.

In some embodiments of the present disclosure, the disease is selected from eye diseases and ear diseases.

In the present disclosure, the methods according to the eighth aspect, the ninth aspect, the tenth aspect, and the eleventh aspect are for non-therapeutic purposes, such as laboratory research for drug discovery and kit development.

A fourteenth aspect of the present disclosure provides a method for treating a disease associated with a mutation in exon 13 of USH2A, comprising administering to a patient in need with an effective amount of the snRNA nucleic acid molecule according to the first aspect, the combination according to the second aspect, the DNA molecule according to the third aspect, the gene expression cassette according to the fourth aspect, the recombinant expression vector according to the fifth aspect, the virus particle according to the sixth aspect, or the pharmaceutical composition according to the seventh aspect.

In some embodiments of the present disclosure, the disease associated with a mutation in exon 13 of USH2A is as described in the thirteenth aspect.

On the basis of common knowledge in the art, the above preferred conditions can be arbitrarily combined to obtain preferred embodiments of the present disclosure.

The reagents and raw materials used in the present disclosure are commercially available.

The positive and progressive effects of the present disclosure are as follows:

The present disclosure provides an snRNA nucleic acid molecule targeting exon 13 of USH2A and flanking target regions thereof. The snRNA nucleic acid molecule comprises a recognition domain, a stem-loop sequence, and an Sm sequence, with the number of recognition domains being at least two. The at least two recognition domains form a "tandem" structure in the snRNA. The tandem of U7-snRNA targeting different target sites can efficiently induce splicing skipping of exon 13 of USH2A, with a higher frequency of single skipping. Especially for pathogenic or non-pathogenic exon 13 of USH2A, the snRNA of the present disclosure can safely and effectively induce single skipping of exon 13 of USH2A at low doses, demonstrating significant clinical value in the prevention and/or treatment of eye diseases and ear diseases associated with abnormal expression of the Usherin protein.

### BRIEF DESCRIPTION OF THE DRAWINGS

FIG. 1 is a schematic diagram showing the structure and effect of U7-snRNA targeting exon 13 of USH2A, using a single recognition domain as an example.
FIG. 2 is a schematic diagram showing the genomic location of U7-snRNA targeting the target region 8.
FIGs. 3A to 3B show the effects of U7 snRNAs targeting different target sites in inducing splicing skipping of exon 13 of USH2A pre-mRNA in reporter gene cells.
FIG. 4 is a schematic diagram showing the efficiency of snRNAs targeting different target sites in effectively inducing single splicing skipping of exon 13 of USH2A pre-mRNA.
FIG. 5 is a schematic diagram showing the genomic location of U7-snRNA targeting the target region 1.
FIG. 6 is a graph showing the proportion of cells in which U7-snRNAs targeting the target region 1 induce splicing skipping of exon 13 of USH2A pre-mRNA in reporter vector cells.
FIG. 7 is a histogram showing the mean FITC intensity of GFP-positive cells induced by U7-snRNA targeting the target region 1 (splicing skipping of exon 13 of USH2A pre-mRNA).
FIG. 8 is a schematic diagram showing the genomic location of U7-snRNA targeting the target region 2.
FIG. 9 is a graph showing the proportion of cells in which U7-snRNAs targeting the target region 2 induce splicing skipping of exon 13 of USH2A pre-mRNA in reporter vector cells.
FIG. 10 is a histogram showing the mean FITC intensity of GFP-positive cells induced by U7-snRNA targeting the target region 2 (splicing skipping of exon 13 of USH2A pre-mRNA).
FIG. 11 is a schematic diagram showing the genomic location of U7-snRNA targeting the target region 3.
FIG. 12 is a graph showing the proportion of cells in which U7-snRNAs targeting the target region 3 induce splicing skipping of exon 13 of USH2A pre-mRNA in reporter vector cells.
FIG. 13 is a histogram showing the mean FITC intensity of GFP-positive cells induced by U7-snRNA targeting the target region 3 (splicing skipping of exon 13 of USH2A pre-mRNA).
FIG. 14 is a schematic diagram showing the genomic location of U7-snRNA targeting the target region 4.
FIG. 15 is a graph showing the proportion of cells in which U7-snRNAs targeting the target region 4 induce splicing skipping of exon 13 of USH2A pre-mRNA in reporter vector cells.
FIG. 16 is a schematic diagram showing the genomic location of U7-snRNA targeting the target region 5.
FIG. 17 is a graph showing the proportion of cells in which U7-snRNAs targeting the target region 5 induce splicing skipping of exon 13 of USH2A pre-mRNA in reporter vector cells.
FIG. 18 is a schematic diagram showing the genomic location of U7-snRNA targeting the target region 6.
FIG. 19 is a graph showing the proportion of cells in which U7-snRNAs targeting the target region 6 induce splicing skipping of exon 13 of USH2A pre-mRNA in reporter vector cells.
FIG. 20 is a schematic diagram showing the genomic location of U7-snRNA targeting the target region 7.
FIG. 21 is a graph showing the proportion of cells in which U7-snRNAs targeting the target region 7 induce splicing skipping of exon 13 of USH2A pre-mRNA in reporter vector cells.
FIG. 22 is a graph showing the mean FITC intensity of cells in which U7 snRNAs targeting different regions induce splicing skipping of exon 13 of USH2A pre-mRNA in reporter gene cells.
FIG. 23 is a schematic diagram showing the structure of tandem U7 snRNA in the present disclosure.
FIG. 24A is a schematic diagram 1 showing the targeting method of tandem U7 snRNA to USH2A pre-mRNA in the present disclosure.
FIG. 24B is a schematic diagram 2 showing the targeting method of tandem U7 snRNA to USH2A pre-mRNA in the present disclosure.
FIG. 25 shows the efficiency of pUC57-U7-snRNA with tandem recognition domains in inducing splicing skipping of exon 13 of USH2A pre-mRNA in Example 8-1.
FIG. 26 shows the efficiency of pUC57-U7-snRNA with tandem recognition domains in inducing splicing skipping of exon 13 of USH2A pre-mRNA in Example 8-2.
FIG. 27 shows the efficiency of chemically synthesized U7 snRNA in inducing splicing skipping of exon 13 of USH2A pre-mRNA in WERI cells;
in which lane 1: 50 pmol chemically synthesized and modified U7-snRNA#30 + #4; lane 2: 50 pmol chemically synthesized and modified U7-snRNA#26 + #15; lane 3: 50 pmol AON1; lane 4: 50 pmol AON2; lane 5: EGFP; lane 6: GL DNA Marker 2000.
FIG. 28 is a histogram showing the quantitative analysis of RT-PCR electrophoresis bands in Example 9;
in which ▲E12-E13 represents USH2A mRNAs with simultaneous splicing skipping of exon 12 and exon 13, and total ▲ represents the sum of USH2A mRNAs with splicing skipping of exon 13 or simultaneous splicing skipping of exon 12 and exon 13.
FIG. 29A is a schematic diagram showing the structure of tandem U7 snRNA.
FIG. 29B shows a comparison of the efficiency of U7-hnRNP A1-snRNA and tandem U7-snRNA in inducing splicing skipping of exon 13 of USH2A pre-mRNA.
FIG. 30 shows that 3×U7 snRNA tandem induces splicing skipping of exon 13 of USH2A, as tested by an *in vitro* gradient escalation assay.
FIG. 31 shows that AAV-U7 snRNA tandem significantly outperforms AON in inducing splicing skipping, as verified by an *in vitro* dose escalation assay.
FIG. 32 shows that snRNA induces splicing skipping of exon 13 of USH2A pre-mRNA in humanized mouse retinal cells more effectively than AON.
FIG. 33 shows that the injection of AAV-U7 snRNA of different serotypes induces splicing skipping of exon 13 of USH2A pre-mRNA in rabbit eye cells more effectively than AON.
FIG. 34 shows that U7 snRNA delivered by AAV can still maintain the induction of splicing skipping of exon 13 of USH2A pre-mRNA after 22 weeks.
FIG. 35 shows the long-term maintenance of efficiency of splicing skipping induced by AAV-1×U7 snRNA tandem and AAV-4×U7 snRNA combination.

### DETAILED DESCRIPTION OF THE PREFERRED EMBODIMENT

The present disclosure is further illustrated below by means of examples, but the present disclosure is not limited to the scope of the examples. The experimental methods for which specific conditions are not indicated in the following examples are selected according to conventional methods and conditions, or according to the product instructions.

### Example 1: Synthesis of U7-snRNA

### 1. Synthesis of snRNA scaffold

Wild-type U7-snRNA comprises a stem-loop structure (scaffold), a U7-specific Sm sequence (AAUUUGUCUAG, SEQ ID NO: 5), and a recognition domain (complementary to replication-dependent histone pre-mRNA). The U7 snRNA of the present disclosure can be derived from the gene sequence of mouse wild-type U7 snRNA (NCBI Reference Sequence: NR_024201.3) from the NCBI database. Specifically, the U7-specific Sm binding site is replaced with an optimized consensus Sm sequence, *i.e.,* SmOPT (AAUUUUUGGAG, SEQ ID NO: 6), the original recognition domain at the 5' end of the SmOPT sequence is replaced with a recognition domain that is reverse complementary to the specific target site of USH2A pre-mRNA, and the original U7 stem-loop structure sequence (CAGGUUUUCUGACUUCGGUCGGAAAACCCCU, SEQ ID NO: 7) is retained at the 3' end of the SmOPT sequence.

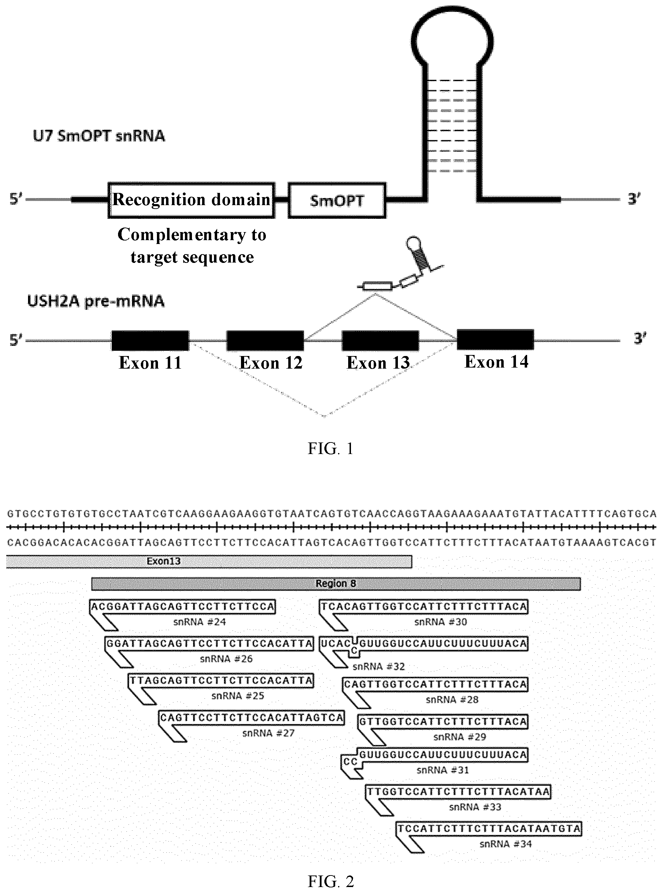

As shown in FIG. 1, the non-tandem U7 snRNA recognition domain sequence targeting exon 13 of USH2Apre-mRNA is reverse complementary to a target sequence selected from intron 12-exon 13-intron 13 of USH2A pre-mRNA. The target sequence can be selected from the target region of the 3' sequence of exon 13 of USH2A pre-mRNA.

The specific operation process is as follows:

First, the pUC57 vector containing the gene sequence: U7-snRNA gene expression cassette scaffold (5'-mouse U7 promoter-smOPT sequence-U7 snRNA scaffold-snRNA gene-specific 3' cassette-3'), was synthesized by means of total gene synthesis. Two Type II restriction enzyme recognition sites (*e.g*., BsaI, AarI, BsmBI) were added between the U7 promoter and smOPT to facilitate subsequent excision, substitution, and insertion of other recognition domain sequences. The snRNA gene-specific 3' cassette was a sequence following the 3' end of the U7 snRNA gene in the mouse genome (GenBank: X54748.1) containing "GTCTACAATGAAA (SEQ ID NO: 8)", which was involved in the processing of pre-snRNA.

### 2. Construction of U7 snRNA vector targeting the target region

The sequence of the 3' region of exon 13 of USH2A pre-mRNA (SEQ ID NO: 1) corresponds to the human genomic location Chr1: 216246563-216246753 (corresponding to the NCBI database version GRch38). The sequence of SEQ ID NO: 1 is as follows:

UAAAUAUAUUUUAUCUUUAGGGCUUAGGUGUGAUCAUUGCAAUUUUG GAUUUAAAUUUCUCCGAAGCUUUAAUGAUGUUGGAUGUGAGCCCUGCCAGUG UAACCUCCAUGGCUCAGUGAACAAAUUCUGCAAUCCUCACUCUGGGCAGUGUG AGUGCAAAAAAGAAGCCAAAGGACUUCAGUGUGACACCUGCAGAGAAAACUU UUAUGGGUUAGAUGUCACCAAUUGUAAGGCCUGUGACUGUGACACAGCUGGA UCCCUCCCUGGGACUGUCUGUAAUGCUAAGACAGGGCAGUGCAUCUGCAAGCC CAAUGUUGAAGGGAGACAGUGCAAUAAAUGUUUGGAGGGAAACUUCUACCUA CGGCAAAAUAAUUCUUUCCUCUGUCUGCCUUGCAACUGUGAUAAGACUGGGAC AAUAAAUGGCUCUCUGCUGUGUAACAAAUCAACAGGACAAUGUCCUUGCAAA UUAGGGGUAACAGGUCUUCGCUGUAAUCAGUGUGAGCCUCACAGGUACAAUU UGACCAUUGACAAUUUUCAACACUGCCAGAUGUGUGAGUGUGAUUCCUUGGG GACAUUACCUGGGACCAUUUGUGACCCAAUCAGUGGCCAGUGCCUGUGUGUGC CUAAUCGUCAAGGAAGAAGGUGUAAUCAGUGUCAACCAGGUAAGAAAGAAAU GUAUUACAU. In addition to the sequences with no mutations listed above, the target regions of exon 13 of USH2A pre-mRNA and flanking target regions (the pre-mRNA region corresponding to Chr1: 216246563-216247246) described above may also be target regions containing natural pathogenic/non-pathogenic mutations, including a target region with at least one of the following mutations: c.2242C>T, c.2276G>T, c.2299delG, c.2522C>A, c.2541C>A, c.2761delC, c.2776C>T, c.2802T>G, c.2209C>T, c.2310delA, c.2391_2392deITG, c.2431A>T, c.2431_2432delAA, c.2440C>T, c.2525dup, c.2610C>A, c.2755C>T, c.2176T>C, c.2236C>G, c.2296T>C, and c.2332G>T.

The sequence of the 5' target region of exon 13 of USH2A pre-mRNA (the pre-mRNA region corresponding to Chr1: 216247130-216247246) (SEQ ID NO: 2) is as follows:

UAAAUAUAUUUUAUCUUUAGGGCUUAGGUGUGAUCAUUGCAAUUUUG
GAUUUAAAUUUCUCCGAAGCUUUAAUGAUGUUGGAUGUGAGCCCUGCCAGUG
UAACCUCCAUGGCUCAGU

The sequence of the 3' target region of exon 13 of USH2A pre-mRNA (the pre-mRNA region corresponding to Chr1: 216246563-216246753) (SEQ ID NO: 3) is as follows:

CAGUGUGAGCCUCACAGGUACAAUUGACCAUUGACAAUUUCAACAC

UGCCAGAUGUGUGAGUGUGAUUCCUUGGGGACAUUACCUGGGACCAUUUGUG
ACCCAAUCAGUGGCCAGUGCCUGUGUGUGCCUAAUCGUCAAGGAAGAAGGUG
UAAUCAGUGUCAACCAGGUAAGAAAGAAAUGUAUUACAU

The sequence of the preferred 3' target region of exon 13 of USH2A pre-mRNA (the pre-mRNA region corresponding to Chr1: 216246563-216246649) (SEQ ID NO: 4) is as follows:

AAUCAGUGGCCAGUGCCUGUGUGUGCCUAAUCGUCAAGGAAGAAGGU
GUAAUCAGUGUCAACCAGGUAAGAAAGAAAUGUAUUACAU

The sequence of the 3' region of exon 13 of USH2A pre-mRNA (the pre-mRNA region corresponding to Chr1: 216246563-216246626) (region 8, SEQ ID NO: 9) is UGCCUAAUCGUCAAGGAAGAAGGUGUAAUCAGUGTCAACCAGGUAAGAAAGA AAUGUAUUACAU, or may be a sequence of the 3' region of exon 13 of USH2A pre-mRNA with natural mutations, such as UGCCUAAUCGUCAAGGAAGAAGGUGUAAUCAGUGGCAACCAGGUAAGAAAGA AAUGUAUUACAU (the underlined portion indicates the natural pathogenic mutation c.2802T>G, SEQ ID NO: 10).

Corresponding Oligo DNAs were synthesized based on the pre-transcriptional DNA sequences corresponding to the snRNA recognition domain sequences listed in Table 1, respectively. The sense strand of the Oligo DNA was the DNA sequence corresponding to the recognition domain sequence, with CCGCA added at 5'. The antisense strand was the reverse complementary sequence of the recognition domain sequence, with AATT added at 5' and T added at 3'. For example, if the recognition domain sequence was 5'-NNN-3', the sense strand of the synthesized Oligo DNA was 5'-CCGCANNN-3', and the antisense strand was 5'-AATTNNNT-3'.

The sense and antisense strands of the synthesized Oligo DNA were mixed according to an annealing reaction system (total reaction volume of 20 µL: 2 µL of Oligo-F (100 µM) + 2 µL of Oligo-R (100 µM) + 2 µL of 10×NEB Cutter smart buffer + 16 µL of deionized water). The mixture was incubated at 95°C for 5 minutes and then cooled on ice to form a double-stranded DNA with sticky ends. After 100-fold dilution, 1 µL of the double-stranded DNA was taken and ligated with 10 ng of linearized pUC57-U7 snRNA backbone plasmid recovered by BsaI digestion using T4 ligase. The ligation product was further transformed into *E. coli* competent cells, then single clones were picked, followed by PCR and sequencing to obtain a U7 snRNA vector for inducing splicing skipping of exon 13 of USH2A. The plasmid was purified and stored at -20°C for later use.

**Table 1: Recognition domain sequences of snRNA**

| snRNA ID # | Recognition domain sequence (5'-3') | SEQ ID | Genomic location corresponding to target sites of USH2A Pre-mRNA | Target region |
|---|---|---|---|---|
| 23 | AUUGUACCUGUGAGGCUCACACUG | 11 | Chr1: 216246730-216246753 | Region 9 |
| 24 | ACCUUCUUCCUUGACGAUUAGGCA | 12 | Chr1: 216246603-216246626 | Region 8 |
| 25 | AUUACACCUUCUUCCUUGACGAUU | 13 | Chr1: 216246598-216246621 | Region 8 |
| 26 | AUUACACCUUCUUCCUUGACGAUUAGG | 14 | Chr1: 216246598-216246624 | Region 8 |
| 27 | ACUGAUUACACCUUCUUCCUUGAC | 15 | Chr1: 216246594-216246617 | Region 8 |
| 28 | ACAUUUCUUUCUUACCUGGUUGAC | 16 | Chr1: 216246570-216246593 | Region 8 |
| 29 | ACAUUUCUUUCUUACCUGGUUG | 17 | Chr1: 216246570-216246591 | Region 8 |
| 30 | ACAUUUCUUUCUUACCUGGUUGACACU | 18 | Chr1: 216246570-216246596 | Region 8 |
| 31 | ACAUUUCUUUCUUACCUGGUUGCC | 19 | Chr1: 216246570-216246593 | Region 8 |
| 32 | ACAUUUCUUUCUUACCUGGUUGCCACU | 20 | Chr1: 216246570-216246596 | Region 8 |
| 33 | AAUACAUUUCUUUCUUACCUGGUU | 21 | Chr1: 216246567-216246590 | Region 8 |
| 34 | AUGUAAUACAUUUCUUUCUUACCU | 22 | Chr1: 216246563-216246586 | Region 8 |
| Scramble | AGGUGUAGUCGACCAUCGUG | 23 | No matching target sequence, negative control | |

snRNA#24, snRNA#25, snRNA#27, and snRNA#29 are homologous in humans and monkeys.

### 3. Chemical synthesis and modification of U7-snRNA

Similar to oligonucleotides, U7 snRNA can also be synthesized by direct chemical synthesis to produce RNA containing the guide sequence, smOPT, and U7 snRNA scaffold. U7 snRNA synthesized *in vitro* can be specifically modified to resist nuclease degradation or enhance affinity for the target sequence.

In this example, U7 snRNA was chemically synthesized, with the three bases at both the 5' and 3' ends each subjected to 2'-O-methyl (2'-OME) modification and phosphorothioate modification to increase nuclease resistance. Taking snRNA#25 and snRNA#26 as examples, the sequences and modifications of the chemically synthesized snRNAs are as follows (* indicates phosphorothioate backbone, m indicates 2'-O-methyl modification, the underlined portion indicates the recognition domain that is reverse complementary to the target sequence, and the italicized portion indicates the smOPT sequence):

The sequence of chemically synthesized and modified U7-snRNA#25 is as follows:

]  5′-
mA*mU*mU*ACACCUUCUUCCUUGACGAUUAAUUUUGGAGCAGGUUUCUGA
CUUCGGUCGGAAAACCmC*mC*mU*-3′ (SEQ ID NO: 24)

The bidirectional extension sequence of chemically synthesized and modified U7-snRNA#25 is as follows:

5′-
mA*mC*mU*GAUUACACCUUCUUCCUUGACGAUUAGGCAAAUUUUUGGAGCAG
GUUUCUGACUUCGGUCGGAAAACCmC*mC*mU*-3′ (SEQ ID NO: 25)

The sequence of chemically synthesized and modified U7-snRNA#26 is as follows:

5′-
mA*mU*mU*ACACCUUCUUCCUUGACGAUUAGGAAUUUUGGAGCAGGUUUUC
UGACUUCGGUCGGAAAACCmC*mC*mU*-3′ (SEQ ID NO: 26)

The unidirectional extension sequence of chemically synthesized and modified U7-snRNA#26 is as follows:

5′-
mA*mU*mU*ACACCUUCUUCCUUGACGAUUAGGCACACACAGGCACAAUUUUUG
GAGCAGGUUUCUGACUUCGGUCGGAAAACCmC*mC*mU*-3′ (SEQ ID NO: 27)

### Example 2: Construction of reporter vector for quantitative evaluation of the efficiency of splicing skipping of exon 13 of USH2A

The RGleft-USH2A Exon13mut-RGright sequence (with AgeI and EcoRI restriction sites added at the 5' and 3' ends, respectively) was obtained by means of total gene synthesis. The synthesized sequence and pX601 plasmid (Addgene, 61591) were digested with restriction enzymes AgeI and EcoRI, followed by gel electrophoresis, gel extraction, and ligation. The synthesized sequence was inserted between the AgeI and EcoRI restriction sites of the pX601 vector to replace the SaCas9 gene sequence of the original vector, resulting in a reporter vector. The reporter vector was further transformed into *E. coli* competent cells, then single clones were picked, followed by PCR and sequencing to obtain a purified reporter plasmid, which was stored at -20°C for later use.

The reporter vector has a structure of pCMV-RGleft-USH2A Exon13mut-RGright, where RG represents a reporter gene, RGleft represents the first half of the 5' end of the reporter gene without a reporting function, and RGright represents the second half of the 3' end of the reporter gene without a reporting function. The tandem expression of RGleft and RGright can normally perform a complete reporter gene function. In the examples of the present disclosure, the reporter gene is the green fluorescent gene EGFP, and the vector has a structure of pCMV-EGFPleft-Exon13mut-EGFPright. Exon13mut represents exon 13 of USH2A containing a pathogenic mutation, as well as upstream and downstream intron sequences thereof (the upstream intron sequence is a tandem gene sequence of 204 bp at the 5' end and 490 bp at the 3' end of intron 12 of the human USH2A gene; the downstream intron sequence is a tandem gene sequence of 703 bp at the 5' end and 216 bp at the 3' end of intron 13 of the human USH2A gene). In the examples of the present disclosure, the pathogenic mutation in exon 13 of USH2A may be c.2299delG, c.2802T>G, or any other mutation, resulting in vector structures of pCMV-EGFPleft-Exon13c.2299delG-EGFPright and pCMV-EGFPleft-Exon13 c.2802T>G-EGFPright. In some examples, the mutation may also be or include c.2276G>T, c.2522C>A, c.2242C>T, c.2541C>A, c.2761delC, c.2776C>T, *etc.*

The sequence of RGleft, such as EGFPleft, is as follows:

atggtgagcaagggcgaggagctgttcaccggggtggtgcccatcctggtcgagctggacggcgacgtaaacggcca
caagttcagcgtgtccggcgagggcgagggcgatgccacctacggcaagctgaccctgaagttcatctgcaccaccggcaagctgc
ccgtgccctggcccaccctcgtgaccaccctgacctacggcgtgcagtgcttcagccgctaccccgaccacatgaagcagcacgact
tcttcaagtccgccatgcccgaaggctacgtccaggagcgcaccatcttcttcaaggacgacggcaactacaagacccgcgccgag
(SEQ ID NO: 28)

The sequence of RGright, such as EGFPright, is as follows:

gtgaagttcgagggcgacaccctggtgaaccgcatcgagctgaagggcatcgacttcaaggaggacggcaacatcctg
gggcacaagctggagtacaactacaacagccacaacgtctatatcatggccgacaagcagaagaacggcatcaaggtgaacttcaa
gatccgccacaacatcgaggacggcagcgtgcagctcgccgaccactaccagcagaacacccccatcggcgacggccccgtgct
gctgcccgacaaccactacctgagcacccagtccgccctgagcaaagaccccaacgagaagcgcgatcacatggtcctgctggagt
tcgtgaccgccgccgggatcactctcggcatggacgagctgtacaagtaa (SEQ ID NO: 29)

### Example 3: Efficiency of splicing skipping of exon 13 of USH2A mediated by U7-snRNAs targeting different target sites in the 3' region (region 8) of exon 13 of USH2A pre-mRNA

293T cells were inoculated into a 24-well plate at a density such that the confluence reached approximately 80% after 24 hours. Lipofectamine 2000 was used to co-transfect 293T cells with pCMV-EGFPleft-Exon13mut-EGFPright and pUC57-U7 snRNA plasmid targeting USH2A pre-mRNA (a vector mass ratio of 100 ng:400 ng). 293T cells transfected with the reporter plasmid alone (Reporter group) and 293T cells co-transfected with the reporter plasmid and pUC57-U7 Scramble (SC group) were used as two negative controls, respectively, while 293T cells without any plasmid transfection were used as a blank control. The transfected cells were cultured for 48 to 72 hours, trypsinized into single cells, and analyzed by flow cytometry to determine the GFP-positive rate (*i.e.,* the proportion of cells in which exon 13 of USH2A was induced to undergo splicing skipping) in different U7 snRNA groups. In this example, the mean FITC intensity (*i.e.,* mean FITC fluorescence intensity of GFP-positive cells) and the GFP-positive rate (*i.e.,* GFP protein expression level of positive cells) were measured in different experimental groups (as shown in FIG. 2).

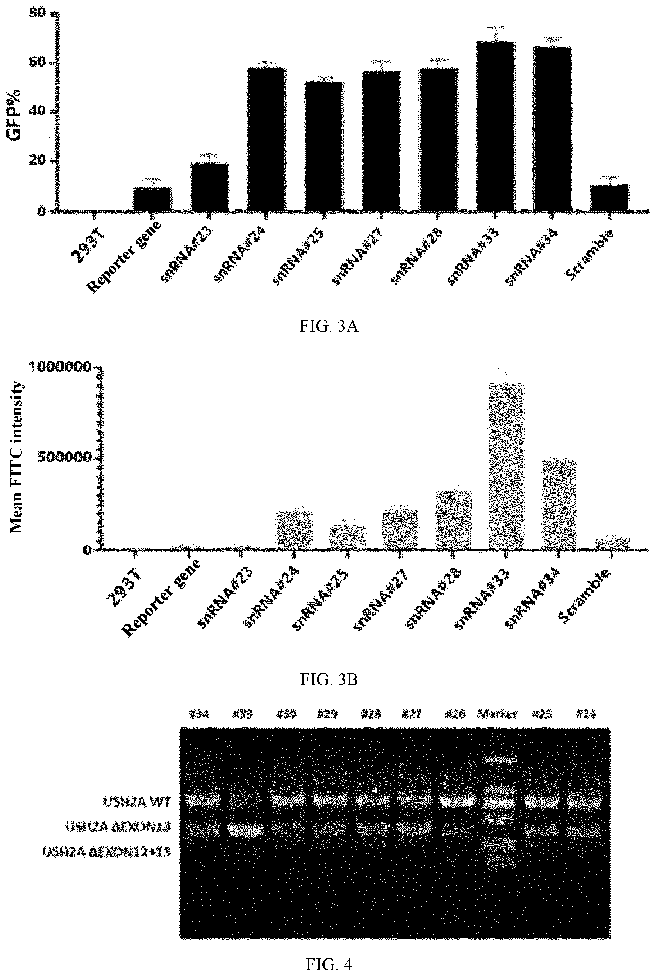

In this example, the efficiency of splicing skipping induced by U7-snRNAs targeting different target sites was compared. Table 2 below and FIGs. 3A to 3B show the effects of U7 snRNAs targeting different target sites in inducing splicing skipping of exon 13 of USH2A pre-mRNAin reporter gene cells. The results demonstrated that all U7-snRNAs (#24 to #34) targeting the 3' sequence of exon 13 of USH2A pre-mRNA were able to induce splicing skipping of exon 13 of USH2A in reporter gene cells with high efficiency.

**Table 2: Proportion of cells in which U7 snRNAs targeting different target sites induce splicing skipping of exon 13 of USH2A pre-mRNA in reporter gene cells**

| Sample name | GFP-positive rate | | | Mean FITC intensity | | |
|---|---|---|---|---|---|---|
| | Round 1 | Round 2 | Round 3 | Round 1 | Round 2 | Round 3 |
| 293T | 0.1% | 0.1% | 0.1% | 6366 | 5100 | 8240 |
| Reporter group | 9.2% | 12.8% | 6.1% | 23925 | 28917 | 17335 |
| snRNA#23 | 18.20% | 23.20% | 16.50% | 21260 | 18898 | 30766 |
| snRNA#24 | 55.70% | 59.10% | 59.30% | 204635 | 198488 | 239915 |
| snRNA#25 | 52.10% | 54.00% | 50.80% | 148755 | 110595 | 163122 |
| snRNA#27 | 52.90% | 61.00% | 55.70% | 222245 | 189021 | 243329 |
| snRNA#28 | 55.90% | 61.80% | 55.90% | 315629 | 283311 | 366659 |
| snRNA#33 | 64.10% | 75.20% | 66.40% | 819508 | 988642 | 918282 |
| snRNA#34 | 65.40% | 70.10% | 64.10% | 494047 | 473049 | 501321 |
| SC group | 11.00% | 13.30% | 7.70% | 58920 | 75279 | 66826 |

### Example 4: Efficient induction of single splicing skipping of exon 13 of USH2A pre-mRNA by chemically synthesized snRNAs

Human host cells were inoculated into a 24-well plate at a density of 6×10⁵ cells/well. In this example, the human retinal nerve cells used were WERI-Rb-1 cells (retinal nerve cell line). 100 pmol of U7-snRNA#24, #25, #26, #27, #28, #29, #30, #33, and #34 synthesized *in vitro* were transfected into WERI cells using Lipofectamine 2000. The transfected cells were cultured for 72 hours. RNA was then extracted from each experimental group and reverse transcribed to obtain cDNA, which was subjected to RT-PCR using primers AGCCTTTCCGCCAAGGTGATC (SEQ ID NO: 30) and CACAACGTTGCCCAGCAATGG (SEQ ID NO: 31) to detect the presence of exon splicing skipping in mature USH2A mRNA. The electrophoresis results are shown in FIG. 4. The results showed that U7-snRNA#24-34 were able to efficiently induce splicing skipping of exon 13, with almost no co-skipping of exon 13 and exon 12 observed. It can be concluded that U7 snRNAs targeting the 3' region can efficiently induce single splicing skipping of exon 13 of USH2A pre-mRNA, demonstrating high safety.

### Example 5: Construction of U7-snRNAs targeting exon 13 of USH2A and different sites near exon 13

In this example, 21 target sites were set for 7 target regions of USH2A pre-mRNA. The 7 target regions of USH2A pre-mRNA are as follows:
Exon 13 region 2 (SEQ ID NO: 33) (Chrl: 216247130-216247161): GAGCCCUGCCAGUGUAACCUCCAUGGCUCAGU;
Exon 13 region 3 (SEQ ID NO: 34) (Chrl: 216246616-216246649): AAUCAGUGGCCAGUGCCUGUGUGUGCCUAAUCGU;
Exon 13 region 4 (SEQ ID NO: 36) (Chrl: 216247210-216247246): UAAAUAUAUUUUAUCUUUAGGGCUUAGGUGUGAUCAU;
Exon 13 region 5 (SEQ ID NO: 37) (Chrl: 216247204-216247232): CUUUAGGGCUUAGGUGUGAUCAUUGCAAU;
Exon 13 region 6 (SEQ ID NO: 38) (Chrl: 216247187-216247220): GGUGUGAUCAUUGCAAUUUUGGAUUUAAAUUUCU;
Exon 13 region 7 (SEQ ID NO: 39) (Chrl: 216247169-216247202): UUGGAUUUAAAUUUCUCCGAAGCUUUAAUGAUGU.

The 21 target sites are shown in the table below.

**Table 3: Recognition domain sequences of snRNA**

| snRNA ID | Recognition domain sequence (5'-3') | SEQ ID | Genomic location corresponding to target sites of USH2A Pre-mRNA | Target region |
|---|---|---|---|---|
| #1 | AGCCCUAAAGAUAAAAUAUAUUUA | 40 | Chr1: 216247223-216247246 | Region 4 |
| #2 | ACCUAAGCCCUAAAGAUAAAAUAU | 41 | Chr1: 216247218-216247241 | Region 4 |
| #3 | AUCACACCUAAGCCCUAAAGAUAA | 42 | Chr1: 216247213-216247236 | Region 4 |
| #4 | AUGAUCACACCUAAGCCCUAAAGAUAA | 43 | Chr1: 216247210-216247236 | Region 4 |
| #5 | AAUGAUCACACCUAAGCCCUAAAG | 44 | Chr1: 216247209-216247232 | Region 5 |
| #6 | AUUGCAAUGAUCACACCUAAGCCC | 45 | Chr1: 216247204-216247227 | Region 5 |
| #7 | AUCCAAAAUUGCAAUGAUCACACC | 46 | Chr1: 216247197-216247220 | Region 6 |
| #8 | AUUUAAAUCCAAAAUUGCAAUGAU | 47 | Chr1: 216247191-216247214 | Region 6 |
| #9 | AGAAAUUUAAAUCCAAAAUUGCAA | 48 | Chr1: 216247187-216247210 | Region 6 |
| #10 | AGCUUCGGAGAAAUUUAAAUCCAA | 49 | Chr1: 216247179-216247202 | Region 7 |
| #11 | AUUAAAGCUUCGGAGAAAUUUAAA | 50 | Chr1: 216247174-216247197 | Region 7 |
| #12 | ACAUCAUUAAAGCUUCGGAGAAAU | 51 | Chr1: 216247169-216247192 | Region 7 |
| #13 | ACAUCCAACAUCAUUAAAGCUUCG | 52 | Chr1: 216247162-216247185 | Region 1 |
| #14 | AGGGCUCACAUCCAACAUCAUUAA | 53 | Chr1: 216247155-216247178 | Region 1 |
| #15 | ACACUGGCAGGGCUCACAUCCA | 54 | Chr1: 216247147-216247168 | Region 1 |
| #16 | ACACUGGCAGGGCUCACAUCCAACAUC | 55 | Chr1: 216247147-216247173 | Region 1 |
| #17 | AGGUUACACUGGCAGGGCUCACAU | 56 | Chr1: 216247142-216247165 | Region 1 |
| #18 | AUGGAGGUUACACUGGCAGGGCUC | 57 | Chr1: 216247138-216247161 | Region 2 |
| #19 | ACUGAGCCAUGGAGGUUACACUGG | 58 | Chr1: 216247130-216247153 | Region 2 |
| #20 | ACACACAGGCACUGGCCACUGAUU | 59 | Chr1: 216246626-216246649 | Region 3 |
| #21 | AGGCACACACAGGCACUGGCCACU | 60 | Chr1: 216246622-216246645 | Region 3 |
| #22 | ACGAUUAGGCACACACAGGCACUG | 61 | Chr1: 216246616-216246639 | Region 3 |

snRNA#9, snRNA#15, snRNA#17, and snRNA#19 are homologous in humans and monkeys.

### Example 6: Detection of efficiency of splicing skipping of exon 13 of USH2A mediated by U7-snRNAs targeting different regions

Following the method described in Example 3, the GFP-positive rate and mean FITC intensity of U7 snRNAs targeting different regions were detected in a reporter cell system.

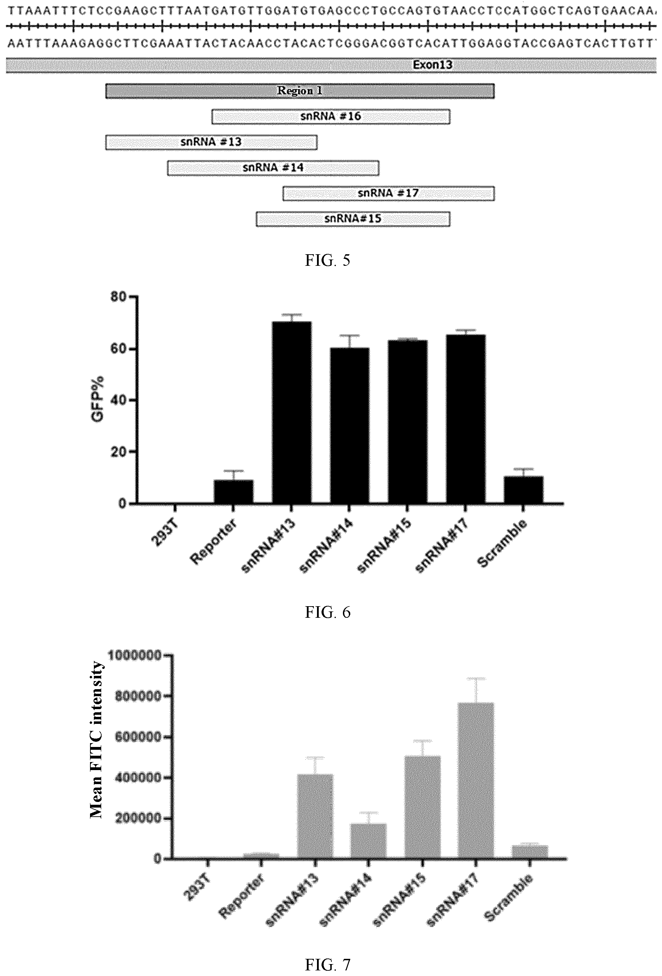

The genomic location of U7-snRNAs targeting the target region 1 (the image from left to right corresponds to the genome from the 5' end to the 3' end) is shown in FIG. 5. The experimental results for U7-snRNAs targeting the target region 1 are shown in FIGs. 6 to 7 and Table 4 below. All U7-snRNAs targeting the target region 1 were able to induce splicing skipping of exon 13 of USH2A in reporter gene cells. In the prior art, AONs targeting the region 1 cannot induce splicing skipping of exon 13, whereas snRNAs targeting the region 1 can efficiently induce splicing skipping of exon 13.

**Table 4: Proportion of cells in which U7-snRNAs targeting the target region 1 induce splicing skipping of exon 13 of USH2A pre-mRNA in reporter gene cells**

| Sample name | GFP-positive rate | | |
|---|---|---|---|
| | Round 1 | Round 2 | Round 3 |
| 293T | 0.1% | 0.1% | 0.1% |
| Reporter | 9.2% | 12.8% | 6.1% |
| snRNA#13 | 71.10% | 67.30% | 72.60% |
| snRNA#14 | 64.80% | 60.80% | 55.20% |
| snRNA#15 | 62.30% | 63.30% | 63.70% |
| snRNA#17 | 65.20% | 67.30% | 64.20% |
| Scramble | 11.00% | 13.30% | 7.70% |

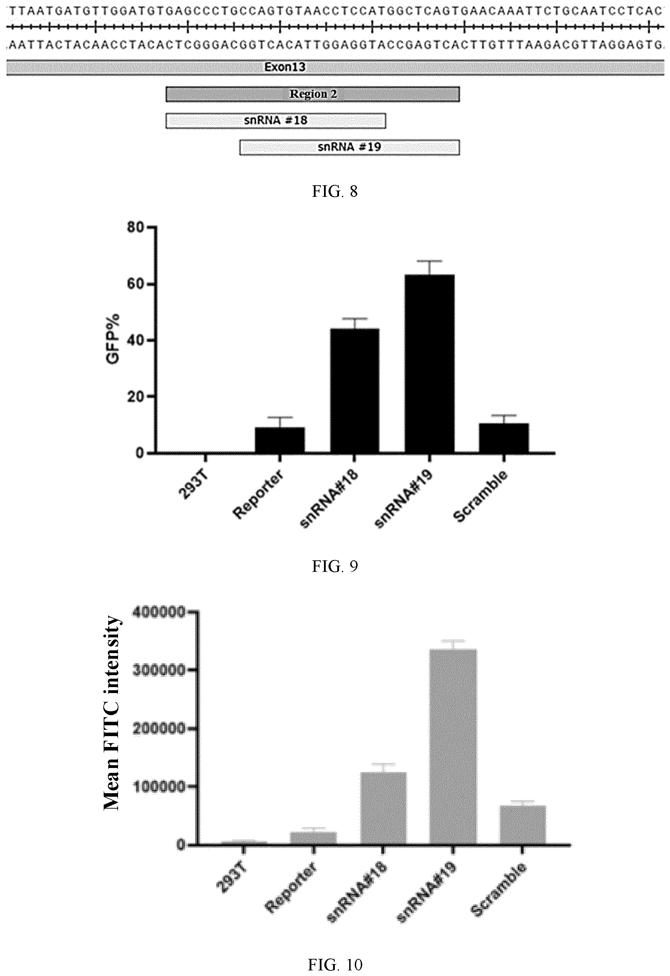

The genomic location of U7-snRNAs targeting the target region 2 (the image from left to right corresponds to the genome from the 5' end to the 3' end) is shown in FIG. 8. The experimental results for U7-snRNAs targeting the target region 2 are shown in FIGs. 9 to 10 and Table 5 below. All U7-snRNAs targeting the target region 2 were able to induce splicing skipping of exon 13 of USH2A in reporter gene cells. In the prior art, AONs targeting the region 2 induce splicing skipping of exon 13 with low efficiency, whereas snRNAs targeting the region 2 can efficiently induce splicing skipping of exon 13.

**Table 5: Proportion of cells in which U7-snRNAs targeting the region 2 induce splicing skipping of exon 13 of USH2A pre-mRNA in reporter gene cells**

| Sample name | GFP-positive rate | | |
|---|---|---|---|
| | Round 1 | Round 2 | Round 3 |
| 293T | 0.1% | 0.1% | 0.1% |
| Reporter | 9.2% | 12.8% | 6.1% |
| snRNA#18 | 43.50% | 47.90% | 40.90% |
| snRNA#19 | 58.50% | 67.80% | 63.80% |
| Scramble | 11.00% | 13.30% | 7.70% |

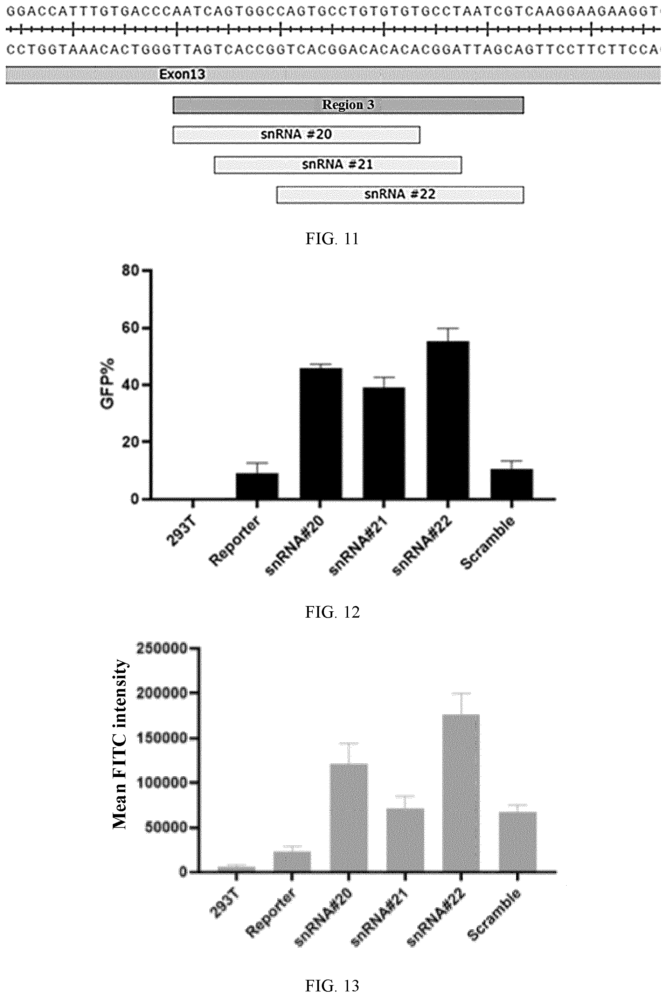

The genomic location of U7-snRNAs targeting the target region 3 (the image from left to right corresponds to the genome from the 5' end to the 3' end) is shown in FIG. 11. The experimental results for U7-snRNAs targeting the target region 3 are shown in FIGs. 12 to 13 and Table 6 below. All U7-snRNAs targeting the target region 3 were able to induce splicing skipping of exon 13 of USH2A in reporter gene cells. In the prior art, AONs targeting the region 3 induce splicing skipping of exon 13 with low efficiency, whereas snRNAs targeting the region 3 can efficiently induce splicing skipping of exon 13.

**Table 6: Proportion of cells in which U7-snRNAs targeting the region 3 induce splicing skipping of exon 13 of USH2A pre-mRNA in reporter gene cells**

| Sample name | GFP-positive rate | | |
|---|---|---|---|
| | Round 1 | Round 2 | Round 3 |
| 293T | 0.1% | 0.1% | 0.1% |
| Reporter | 9.2% | 12.8% | 6.1% |
| snRNA#20 | 44.70% | 47.40% | 45.80% |
| snRNA#21 | 43.10% | 36.40% | 37.80% |
| snRNA#22 | 50.40% | 56.50% | 59.10% |
| Scramble | 11.00% | 13.30% | 7.70% |

In combination with the results for target regions 1, 2, and 3, it was found that although target regions 1, 2, and 3 are non-sensitive to AONs, as shown in the prior art, *i.e.,* AONs targeting these regions are unable to induce or inefficiently induce splicing skipping of exon 13, snRNAs targeting these regions can significantly induce splicing skipping of exon 13. Thus, although both snRNAs and AONs can induce splicing skipping, their mechanisms of action and sensitivity to target sites (applicable target regions and target sites) are different.

Meanwhile, as shown in FIGs. 6, 9, 12, and Table 7 below, although the GFP% (proportion of cells with induced splicing skipping) is similar for snRNAs targeting the same region, the mRNA and protein levels (mean FITC intensity) for splicing skipping induced by snRNAs targeting different target sites of the same region vary in the same cells. Moreover, the mRNA and protein levels for splicing skipping induced by snRNAs targeting the target region 1 and target region 2 in the same cells are superior to those induced by snRNAs targeting the target region 3.

**Table 7: Mean FITC intensity of GFP-positive cells induced by U7-snRNAs targeting the target regions 1, 2, and 3 (splicing skipping of exon 13 of USH2A pre-mRNA)**

| Sample name | Mean FITC intensity | | |
|---|---|---|---|
| | Round 1 | Round 2 | Round 3 |
| 293T | 6366 | 5100 | 8240 |
| Reporter | 23925 | 28917 | 17335 |
| snRNA#13 | 486351 | 326693 | 437187 |
| snRNA#14 | 232257 | 158859 | 132083 |
| snRNA#15 | 574041 | 428688 | 521495 |
| snRNA#17 | 895368 | 656932 | 746482 |
| snRNA#18 | 141587 | 113914 | 118049 |
| snRNA#19 | 348154 | 321381 | 340581 |
| snRNA#20 | 127942 | 94695 | 139515 |
| snRNA#21 | 83206 | 57570 | 75303 |
| snRNA#22 | 191259 | 150479 | 188522 |
| Scramble | 58920 | 75279 | 66826 |

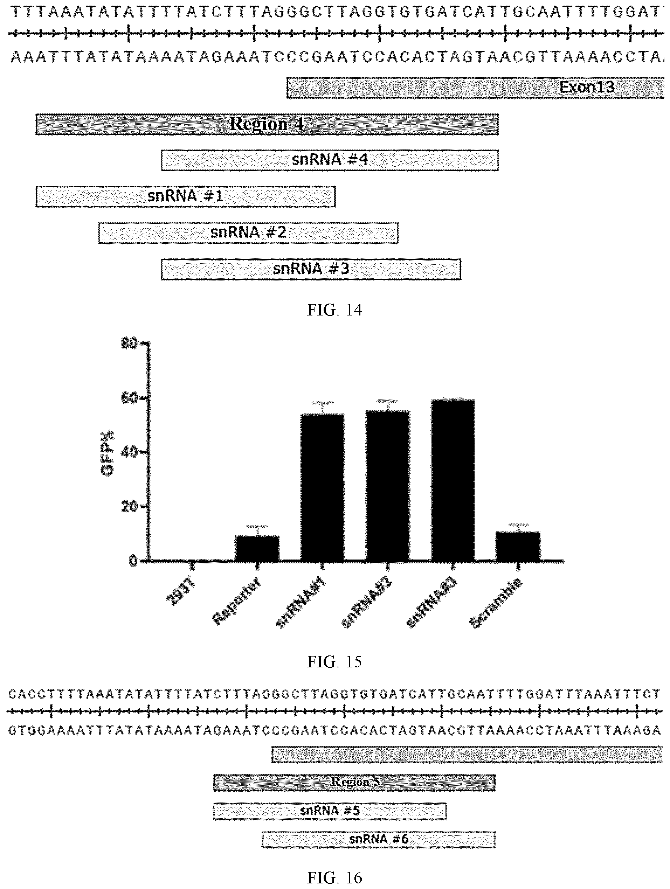

The genomic location of U7-snRNAs targeting the region 4 (the image from left to right corresponds to the genome from the 5' end to the 3' end) is shown in FIG. 14. The experimental results for U7-snRNAs targeting the region 4 are shown in FIG. 15 and Table 8 below. All U7-snRNAs targeting the target region 4 were able to induce splicing skipping of exon 13 of USH2A in reporter gene cells.

**Table 8: Proportion of cells in which U7-snRNAs targeting the region 4 induce splicing skipping of exon 13 of USH2A pre-mRNA in reporter gene cells**

| Sample name | GFP-positive rate | | |
|---|---|---|---|
| | Round 1 | Round 2 | Round 3 |
| 293T | 0.10% | 0.10% | 0.10% |
| Reporter | 9.20% | 12.80% | 6.10% |
| snRNA#1 | 50.30% | 58.70% | 52.50% |
| snRNA#2 | 53.20% | 59.40% | 52.90% |
| snRNA#3 | 59.00% | 59.90% | 58.70% |
| Scramble | 11.00% | 13.30% | 7.70% |

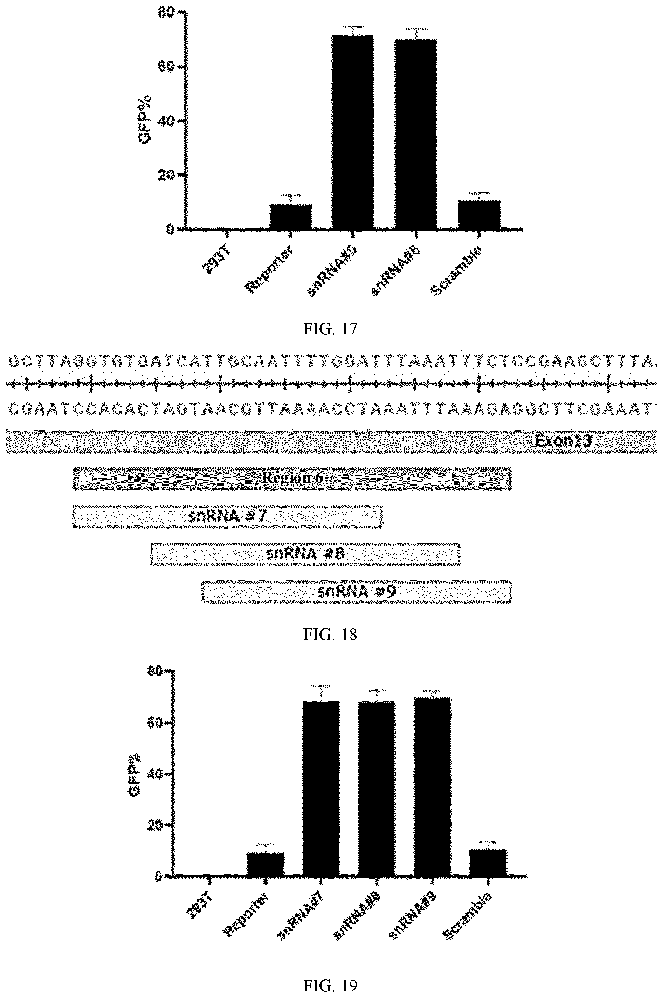

The genomic location of U7-snRNAs targeting the target region 5 (the image from left to right corresponds to the genome from the 5' end to the 3' end) is shown in FIG. 16. The experimental results for U7-snRNAs targeting the target region 5 are shown in FIG. 17 and Table 9 below. All U7-snRNAs targeting the target region 5 were able to induce splicing skipping of exon 13 of USH2A in reporter gene cells.

**Table 9: Proportion of cells in which U7-snRNAs targeting the region 5 induce splicing skipping of exon 13 of USH2A pre-mRNA in reporter gene cells**

| Sample name | GFP-positive rate | | |
|---|---|---|---|
| | Round 1 | Round 2 | Round 3 |
| 293T | 0.1% | 0.1% | 0.1% |
| Reporter | 9.2% | 12.8% | 6.1% |
| snRNA#5 | 67.70% | 74.30% | 72.10% |
| snRNA#6 | 67.90% | 67.80% | 74.60% |
| Scramble | 11.00% | 13.30% | 7.70% |

The genomic location of U7-snRNAs targeting the target region 6 (the image from left to right corresponds to the genome from the 5' end to the 3' end) is shown in FIG. 18. The experimental results for U7-snRNAs targeting the target region 6 are shown in FIG. 19 and Table 10 below. All U7-snRNAs targeting the target region 6 were able to induce splicing skipping of exon 13 of USH2A in reporter gene cells.

**Table 10: Proportion of cells in which U7-snRNAs targeting the region 6 induce splicing skipping of exon 13 of USH2A pre-mRNA in reporter gene cells**

| Sample name | GFP-positive rate | | |
|---|---|---|---|
| | Round 1 | Round 2 | Round 3 |
| 293T | 0.10% | 0.10% | 0.10% |
| Reporter | 9.20% | 12.80% | 6.10% |
| snRNA#7 | 71.40% | 61.30% | 72.20% |
| snRNA#8 | 64.50% | 73.10% | 66.50% |
| snRNA#9 | 66.50% | 70.80% | 71.10% |
| Scramble | 11.00% | 13.30% | 7.70% |

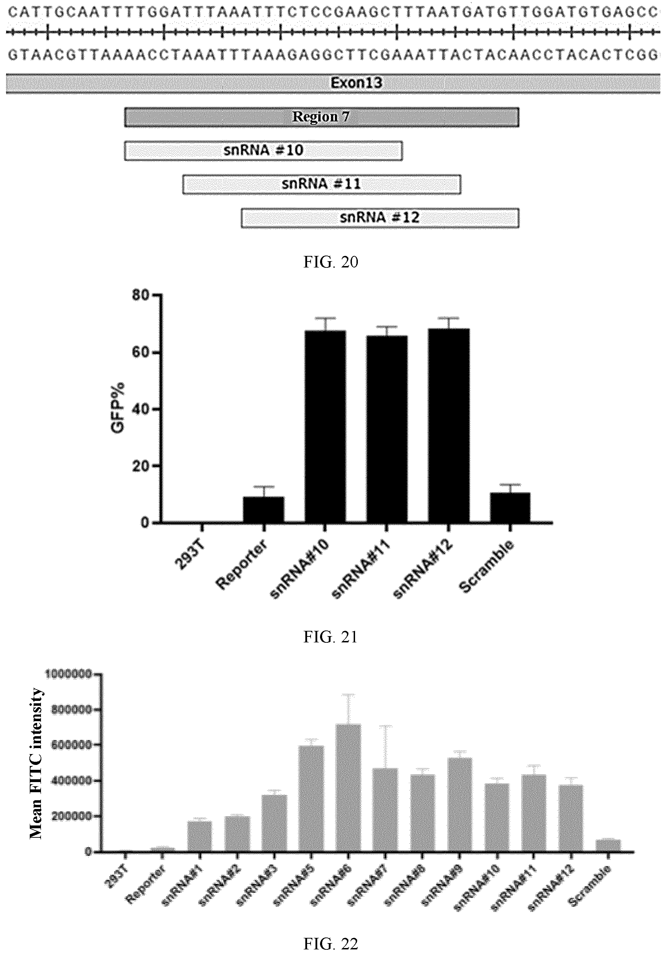

The genomic location of U7-snRNAs targeting the target region 7 (the image from left to right corresponds to the genome from the 5' end to the 3' end) is shown in FIG. 20. The experimental results for U7-snRNAs targeting the target region 7 are shown in FIG. 21 and Table 11 below. All U7-snRNAs targeting the target region 7 were able to induce splicing skipping of exon 13 of USH2A in reporter gene cells.

**Table 11: Proportion of cells in which U7-snRNAs targeting the region 7 induce splicing skipping of exon 13 of USH2A pre-mRNA in reporter gene cells**

| Sample name | GFP-positive rate | | |
|---|---|---|---|
| | Round 1 | Round 2 | Round 3 |
| 293T | 0.10% | 0.10% | 0.10% |
| Reporter | 9.20% | 12.80% | 6.10% |
| snRNA#10 | 62.50% | 69.80% | 70.30% |
| snRNA#11 | 62.90% | 64.70% | 69.30% |
| snRNA#12 | 65.80% | 67.00% | 72.40% |
| Scramble | 11.00% | 13.30% | 7.70% |

The mean FITC intensity of GFP-positive cells induced by U7-snRNAs targeting different regions is shown in FIG. 22 and Table 12 below. Although the GFP% (proportion of cells with induced splicing skipping) is similar for snRNAs targeting the same region, the mRNA and protein levels (mean FITC intensity) for splicing skipping induced by snRNAs targeting different target sites of the same region vary in the same cells.

Targeting the region 2 not only resulted in a higher proportion of cells with induced splicing skipping (GFP%), but also exhibited high mRNA and protein levels (mean FITC intensity) for splicing skipping by induction in the same cells.

In the prior art, target site #2 and its adjacent sites #1 and #3 (region 4) with high efficiency of splicing skipping induced by AONs exhibit low mRNA levels for splicing skipping by induction in the same cells in the snRNA system. In the prior art, targeting the region 7 is more efficient than targeting the region 5 for AONs, but in the snRNA system, targeting the region 5 is more efficient than targeting the region 7. In the prior art, targeting the region 3 is more efficient than targeting the region 2 for AONs, but in the snRNA system, targeting the region 2 is more efficient than targeting the region 3. Thus, although both snRNAs and AONs can induce splicing skipping, their mechanisms of action and sensitivity to target sites are different. In combination with the results of Example 8, since the efficiency of snRNA#24 is comparable to that of snRNA#2 and AON1 (SEQ ID NO: 77), it can be inferred that the effects of snRNA#3 to #11 are all superior to those of snRNA#2, snRNA#24, and AON1.

**Table 12: Mean FITC intensity of cells in which U7 snRNAs targeting different regions induce splicing skipping of exon 13 of USH2A pre-mRNA in reporter gene cells**

| Sample name | Mean FITC intensity | | |
|---|---|---|---|
| | Round 1 | Round 2 | Round 3 |
| 293T | 6366 | 5100 | 8240 |
| Reporter | 23925 | 28917 | 17335 |
| snRNA#1 | 173453 | 155132 | 187790 |
| snRNA#2 | 206250 | 189836 | 202251 |
| snRNA#3 | 326133 | 290451 | 341559 |
| snRNA#5 | 597519 | 630198 | 554593 |
| snRNA#6 | 860189 | 533418 | 759756 |
| snRNA#7 | 635438 | 192090 | 573484 |
| snRNA#8 | 464705 | 437438 | 403231 |
| snRNA#9 | 506956 | 570141 | 504600 |
| snRNA#10 | 416595 | 374370 | 358388 |
| snRNA#11 | 485513 | 430752 | 384956 |
| snRNA#12 | 422896 | 346341 | 361230 |
| Scramble | 58920 | 75279 | 66826 |

The U7-snRNAs for inducing splicing skipping of exon 13 of USH2A pre-mRNA in present disclosure are not limited to the U7-snRNAs listed in Examples 3 and 4. The target sites recognized by the recognition domains of the U7-snRNAs in present disclosure are selected from intron 12-exon 13-intron 13 of USH2A pre-mRNA, preferably from exon 13 and flanking target regions (SEQ ID NO: 1).

### Example 7: Splicing skipping of exon 13 of USH2A mediated by U7-snRNA combinations targeting different target sites

### 1. Construction of U7-snRNA multi-target combination vector

According to the Golden Gate Assembly technique, the U7 snRNA cassette (expression cassette) was amplified by PCR using different U7 snRNA plasmids as templates while introducing additional 5' flanking bases and BsaI restriction sites in the correct orientation at both ends of the amplicon using primers, allowing that the adjacent different U7 snRNA cassettes were digested with BsaI to generate specific and complementary sticky ends, and the first and last U7 snRNA cassettes were digested with BsaI to generate the same sticky ends as the linearized backbone vector digested by HindIII + NotI. Finally, the above PCR products were assembled with pUC57-U7 snRNA Backbone recovered by HindIII + NotI digestion using NEB^{®} Golden Gate Assembly Kit (BsaI-HF^{®}v2) (NEB#E1601). The assembly method is as follows: pUC57-U7 snRNA Backbone-HindIII + NotI, 80 ng; U7 snRNA#A cassette PCR product, 20 ng; U7 snRNA#B cassette PCR product, 20 ng; U7 snRNA#C cassette PCR product, 20 ng; T4 DNA Ligase Buffer (10X), 2 µL; NEB Golden Gate Assembly Mix, 1 µL; reaction process: (37°C, 5 minutes → 16°C, 5 minutes) × 20 → 60°C, 5 minutes.

The Golden Gate Assembly product was further transformed into *E. coli* competent cells, then single clones were picked, followed by PCR and sequencing to obtain the U7 snRNA multi-target combination vector for inducing splicing skipping of exon 13 of USH2A. The plasmid was purified and stored at -20°C for later use.

### Example 8-1: Induction of splicing skipping of exon 13 of USH2A in reporter gene cells by U7 snRNA with tandem recognition domains

### 1. Preparation of U7 snRNA with tandem recognition domains

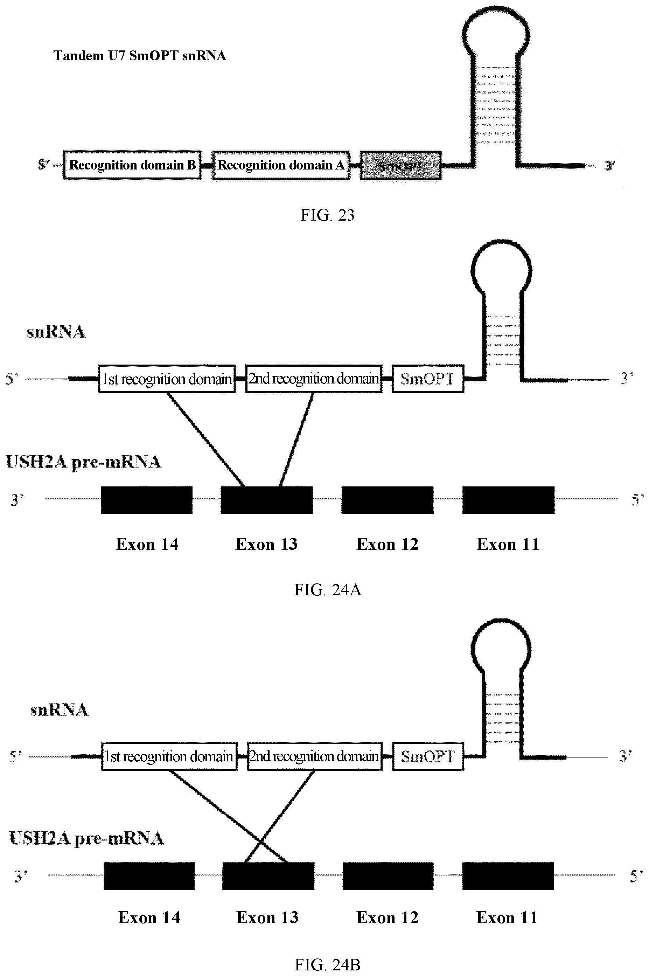

U7 snRNA with tandem recognition domains is defined as a U7 snRNA stem-loop structure and an SmOPT sequence linking two or more recognition domains, with a structure of 5'-recognition domain B-recognition domain A-SmOPT sequence-stem-loop structure-3', as shown in FIG. 23. The recognition domains A and B of the tandem U7 snRNA recognize RNA sequences at different target sites.

Corresponding Oligo DNAs were synthesized based on the pre-transcriptional DNA sequences corresponding to the snRNA recognition domain sequences listed in Tables 1 and 3, respectively. The sense strand of the Oligo DNA was the DNA corresponding to the recognition domain sequence, with CCGCA added at 5'. The antisense strand was the reverse complementary sequence of the recognition domain sequence, with AATT added at 5' and T added at 3'. For example, if the tandem recognition domains of snRNA#15 and snRNA#25 were ACACUGGCAGGGCUCACAUCCA (SEQ ID NO: 54) and AUUACACCUUCUUCCUUGACGAUU (SEQ ID NO: 13), respectively, the sense strand of the synthesized Oligo DNA was *CCGCA*ATTACACCTTCTTCCTTGACGATTACACTGGCAGGGCTCACATCCAAC (SEQ ID NO: 75), and the antisense strand was *AATT*GTTGGATGTGAGCCCTGCCAGTGT-AATCGTCAAGGAAGAAGGTGTAAT*T* (SEQ ID NO: 76).

The sense and antisense strands of the synthesized Oligo DNA were mixed according to an annealing reaction system (total reaction volume of 20 µL: 2 µL of Oligo-F (100 µM) + 2 µL of Oligo-R (100 µM) + 2 µL of 10×NEB Cutter smart buffer + 16 µL of deionized water). The mixture was incubated at 95°C for 5 minutes and then cooled on ice to form a double-stranded DNA with sticky ends. After 100-fold dilution, 1 µL of the double-stranded DNA was taken and ligated with 10 ng of linearized pUC57-U7 snRNA backbone plasmid recovered by BsaI digestion using T4 ligase. The ligation product was further transformed into *E. coli* competent cells, then single clones were picked, followed by PCR and sequencing to obtain a U7 snRNA vector for inducing splicing skipping of exon 13 of USH2A. The plasmid was purified and stored at -20°C for later use. The constructed vector was named pUC57-U7 snRNA#B-#A, where A and B represent the recognition domain numbers, corresponding to the recognition domain lists and sequences in Tables 1 and 3, respectively. For example, pUC57-snRNA#25-#15, pUC57-snRNA#24-#9, pUC57-snRNA#24-#19, pUC57-snRNA#25-#9, pUC57-snRNA#25-#19, pUC57-snRNA#29-#9, and pUC57-snRNA#25-#15.

The U7 snRNA with tandem recognition domains can also be chemically synthesized and modified following the method described in Example 2. For example, the specific sequences and modifications are as follows (* indicates phosphorothioate backbone, m indicates 2'-O-methyl modification, the underlined portion indicates the recognition domain that is reverse complementary to the target sequence, and the italicized portion indicates the smOPT sequence):

Chemically synthesized and modified U7 snRNA#28-#3:

5′-mA*mC*mA*UUUCUUUCUUACCUGGUUGAC-
AUCACACCUAAGCCCUAAAGAUAAAAUUUUUGGAGCAGGUUUCUGACUUCGG
UCGGAAAACCmC*mC*mU*-3′ (SEQ ID NO: 63)

Chemically synthesized and modified U7 snRNA#30-#4:

5′-mA*mA*mC*AUUUCUUUCUUACCUGGUUGACACU-
AUGAUCACACCUAAGCCCUAAAGAUAAAAUUUUUGGAGCAGGUUUUCUGACUU
CGGUCGGAAAACCmC*mC*mU*-3′ (SEQ ID NO: 64)

Chemically synthesized and modified U7 snRNA#25-#15:

5′-mA*mU*mU*ACACCUUCUUCCUUGACGAUU-
ACACUGGCAGGGCUCACAUCCAAAUUUUUGGAGCAGGUUUCUGACUUCGGUC

GGAAAACCmC*mC*mU*-3′ (SEQ ID NO: 65)

Chemically synthesized and modified U7 snRNA#26-#16:

5′-mA*mA*mU*UACACCUUCUUCCUUGACGAUUAGG-
ACACUGGCAGGGCUCACAUCCAACAUCAAUUUUUGGAGCAGGUUUUCUGACUU
CGGUCGGAAAACCmC*mC*mU*-3′ (SEQ ID NO: 66)

In the above sequences, the "-" only indicates the order of linkage between two sequences.

In some examples, the total length of the chemically synthesized snRNA sequence is preferably greater than or equal to 96 bp.

2. Induction of splicing skipping of exon 13 of USH2A in reporter gene cells by U7 snRNA with tandem recognition domains

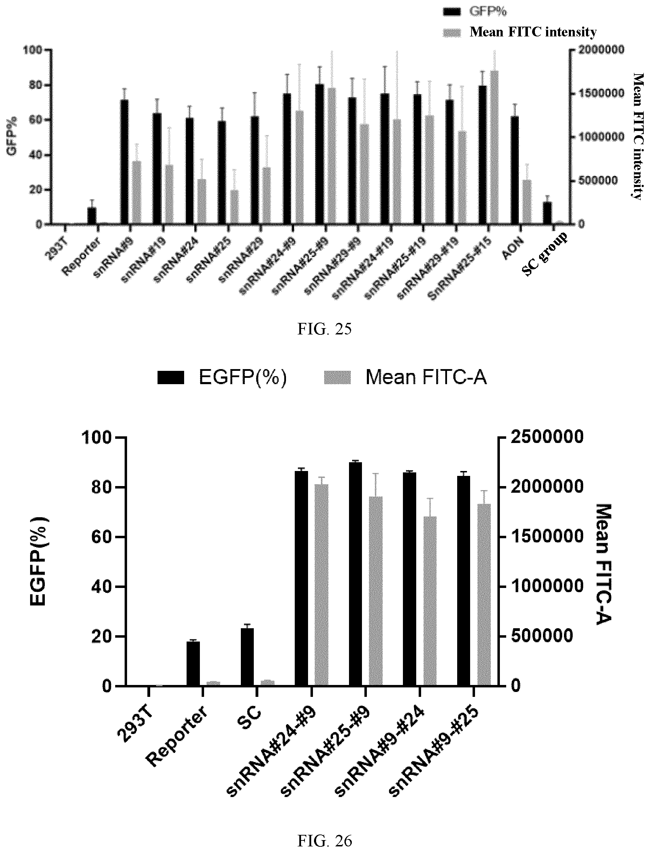

293T cells were inoculated into a 24-well plate at a density such that the confluence reached approximately 80% after 24 hours. Lipofectamine 2000 was used to co-transfect 293T cells with pCMV-EGFPleft-Exon13mut-EGFPright and plasmids expressing U7 snRNA with tandem recognition domains or plasmids expressing U7 snRNA with a single recognition domain, respectively (a vector mass ratio of 100 ng:400 ng). 293T cells transfected with the reporter plasmid alone (Reporter group) and 293T cells co-transfected with the reporter plasmid and pUC57-U7 Scramble (SC group) were used as two negative controls, respectively, while 293T cells without any plasmid transfection were used as a blank control. The transfected cells were cultured for 48 to 72 hours, trypsinized into single cells, and analyzed by flow cytometry to determine the GFP-positive rate in different snRNA groups. Table 13 below and FIG. 25 show the efficiency of tandem U7-snRNAs targeting different target sites in inducing splicing skipping of exon 13 of USH2A pre-mRNA.

In this example, by applying tandem U7-snRNAs targeting different target sites, it was found that the efficiency of inducing splicing skipping of exon 13 of USH2A can be improved in reporter gene cells. In this example, the efficiency of splicing skipping induced by different target sites in tandem is higher than that induced by a single target site and is superior to the known technique of splicing skipping of exon 13 of USH2A. Meanwhile, in combination with the data from other examples, it is surprising that although the efficiency of U7 snRNA#25 alone in inducing splicing skipping of exon 13 was not high, the efficiency of U7 snRNA#25 in tandem with other U7 snRNAs in inducing splicing skipping of exon 13 was significantly improved. Additionally, in the present disclosure, tandem AONs targeting different target sites were constructed and attempted to induce splicing skipping of exon 13 of USH2A pre-mRNA. However, almost no efficiency of splicing skipping was observed, further confirming that the mechanisms of action of AONs and snRNAs in inducing splicing skipping are different.

**Table 13: Efficiency of tandem U7-snRNAs targeting different target sites in inducing splicing skipping of exon 13 of USH2A pre-mRNA**

| Sample name | GFP-positive rate | | | Mean FITC intensity | | |
|---|---|---|---|---|---|---|
| | Round 1 | Round 2 | Round 3 | Round 1 | Round 2 | Round 3 |
| 293T | 0.10% | 0.10% | 0.10% | 5960 | 6100 | 11052 |
| Reporter group | 5.10% | 11.00% | 13.50% | 19637 | 18264 | 25099 |
| snRNA#9 | 68.00% | 78.90% | 67.30% | 537794 | 705890 | 934080 |
| snRNA#19 | 56.00% | 71.50% | 64.80% | 330972 | 544705 | 1166147 |
| snRNA#24 | 54.80% | 67.90% | 61.30% | 299833 | 495247 | 762459 |
| snRNA#25 | 52.00% | 66.60% | 60.00% | 200472 | 337127 | 656348 |
| snRNA#29 | 49.30% | 76.10% | 61.20% | 249725 | 969651 | 742796 |
| snRNA#24-#9 | 62.70% | 84.30% | 78.10% | 757993 | 1821081 | 1338597 |
| snRNA#25-#9 | 69.80% | 89.70% | 81.90% | 970879 | 2081647 | 1637319 |
| snRNA#29-#9 | 62.60% | 84.00% | 72.70% | 806176 | 1748126 | 900419 |
| snRNA#24-#19 | 60.30% | 91.00% | 74.60% | 548209 | 2100852 | 961757 |
| snRNA#25-#19 | 66.70% | 78.80% | 79.10% | 793055 | 1522911 | 1431606 |
| snRNA#29-#19 | 64.60% | 81.10% | 69.30% | 764571 | 1665386 | 773731 |
| snRNA#25-#15 | 76.2% | 89.0% | 74.1% | 1630576 | 2314443 | 1341442 |
| AON | 56.70% | 69.90% | 59.20% | 473261 | 708438 | 342562 |
| SC group | 8.60% | 14.40% | 15.40% | 37615 | 27557 | 41557 |

In this example, RNA sequences recognizing different target sites were linked in tandem in the recognition region of U7 snRNA to construct tandem U7 snRNAs targeting different target sites. The snRNA with tandem recognition domains in this example contains two or more recognition domains in tandem at the 5' end of the snRNA (as shown in FIG. 23). In some examples, two tandem recognition domains can recognize the same target site, thereby increasing the number of targeting recognition domains and enhancing the efficiency of inducing splicing skipping of exon 13 of USH2A pre-mRNA when driven by the same expression vector or the same snRNA.

When the U7 snRNA contains two or more different recognition domains in tandem, the recognition domains from the 5' end to the 3' end of the U7 snRNA are ordered from the 3' end to the 5' end based on the positions of the target sites corresponding to the recognition domains in the USH2A pre-mRNA.

### Example 8-2: Induction of splicing skipping of exon 13 of USH2A in reporter gene cells by U7 snRNA with tandem recognition domains

### 1. Preparation of U7 snRNA with tandem recognition domains

U7 snRNA with tandem recognition domains is defined as a U7 snRNA stem-loop structure and an SmOPT sequence linking two or more recognition domains, with a structure of 5'-recognition domain B-recognition domain A-SmOPT sequence-stem-loop structure-3', as shown in FIG. 23. The recognition domains A and B of the tandem U7 snRNA recognize RNA sequences at different target sites. Furthermore, the targeting method of U7 snRNA with tandem recognition domains to USH2A pre-mRNA is shown in FIG. 24A or FIG. 24B.

Corresponding Oligo DNAs were synthesized based on the pre-transcriptional DNA sequences corresponding to the snRNA recognition domain sequences listed in Tables 1 and 3, respectively. The sense strand of the Oligo DNA was the DNA corresponding to the recognition domain sequence, with CCGCA added at 5'. The antisense strand was the reverse complementary sequence of the recognition domain sequence, with AATT added at 5' and T added at 3'. The method is the same as Example 8-1. For example, if the tandem recognition domains of snRNA#15 and snRNA#25 were ACACUGGCAGGGCUCACAUCCA (SEQ ID NO: 54) and AUUACACCUUCUUCCUUGACGAUU (SEQ ID NO: 13), respectively, the sense strand of the synthesized Oligo DNA was *CCGCA*ATTACACCTTCTTCCTTGACGATTACACTGGCAGGGCTCACATCCAAC (SEQ ID NO: 75), and the antisense strand was *AATT*GTTGGATGTGAGCCCTGCCAGTGT-AATCGTCAAGGAAGAAGGTGTAAT*T* (SEQ ID NO: 76).

The sense and antisense strands of the synthesized Oligo DNA were mixed according to an annealing reaction system (total reaction volume of 20 µL: 2 µL of Oligo-F (100 µM) + 2 µL of Oligo-R (100 µM) + 2 µL of 10×NEB Cutter smart buffer + 16 µL of deionized water). The mixture was incubated at 95°C for 5 minutes and then cooled on ice to form a double-stranded DNA with sticky ends. After 100-fold dilution, 1 µL of the double-stranded DNA was taken and ligated with 10 ng of linearized pUC57-U7 snRNA backbone plasmid recovered by BsaI digestion using T4 ligase. The ligation product was further transformed into *E. coli* competent cells, then single clones were picked, followed by PCR and sequencing to obtain a U7 snRNA vector for inducing splicing skipping of exon 13 of USH2A. The plasmid was purified and stored at -20°C for later use. The constructed vector was named pUC57-U7 snRNA#B-#A, where A and B represent the recognition domain numbers, corresponding to the recognition domain lists and sequences in Tables 1 and 3, respectively. For example, pUC57-snRNA#24-#9, pUC57-snRNA#25-#9, pUC57-snRNA#9-#24, and pUC57-snRNA#9-#25. In pUC57-snRNA#24-#9 and pUC57-snRNA#25-#9, the targeting method of U7 snRNA to USH2A pre-mRNA is shown in FIG. 24A. In pUC57-snRNA#9-#24 and pUC57-snRNA#9-#25, the targeting method of U7 snRNA to USH2A pre-mRNA is shown in FIG. 24B.

The U7 snRNA with tandem recognition domains can also be chemically synthesized and modified following the method described in Example 2, which is the same as Example 8-1. For example, the specific sequences and modifications are as follows (* indicates phosphorothioate backbone, m indicates 2'-O-methyl modification, the underlined portion indicates the recognition domain that is reverse complementary to the target sequence, and the italicized portion indicates the smOPT sequence):

Chemically synthesized and modified U7 snRNA#28-#3:

5′-mA*mC*mA*UUUCUUUCUUACCUGGUUGAC-
AUCACACCUAAGCCCUAAAGAUAAAAUUUUUGGAGCAGGUUUCUGACUUCGG
UCGGAAAACCmC*mC*mU*-3′ (SEQ ID NO: 63)

Chemically synthesized and modified U7 snRNA#30-#4:

5′-mA*mA*mC*AUUUCUUUCUUACCUGGUUGACACU-
AUGAUCACACCUAAGCCCUAAAGAUAAAAUUUUUGGAGCAGGUUUUCUGACUU
CGGUCGGAAAACCmC*mC*mU*-3′ (SEQ ID NO: 64)

Chemically synthesized and modified U7 snRNA#25-#15:

5′-mA*mU*mU*ACACCUUCUUCCUUGACGAUU-
ACACUGGCAGGGCUCACAUCCAAAUUUUGGAGCAGGUUUCUGACUUCGGUC
GGAAAACCmC*mC*mU*-3′ (SEQ ID NO: 65)

Chemically synthesized and modified U7 snRNA#26-#16:

5′-mA*mA*mU*UACACCUUCUUCCUUGACGAUUAGG-
ACACUGGCAGGGCUCACAUCCAACAUCAAUUUUGGAGCAGGUUUCUGACUU

CGGUCGGAAAACCmC*mC*mU*-3′ (SEQ ID NO: 66)

In the above sequences, the "-" only indicates the order of linkage between two sequences.

In some examples, the total length of the chemically synthesized snRNA sequence is preferably greater than or equal to 96 bp.

### 2. Induction of splicing skipping of exon 13 of USH2A in reporter gene cells by U7 snRNA with tandem recognition domains

293T cells were inoculated into a 24-well plate at a density such that the confluence reached approximately 80% after 24 hours. Lipofectamine 2000 was used to co-transfect 293T cells with pCMV-EGFPleft-Exon13mut-EGFPright and plasmids expressing U7 snRNA with tandem recognition domains (a vector mass ratio of 100 ng:400 ng). 293T cells transfected with the reporter plasmid alone (Reporter group) and 293T cells co-transfected with the reporter plasmid and pUC57-U7 Scramble (SC group) were used as two negative controls, respectively, while 293T cells without any plasmid transfection were used as a blank control. The transfected cells were cultured for 48 to 72 hours, trypsinized into single cells, and analyzed by flow cytometry to determine the GFP-positive rate in different snRNA groups. Table 14 below and FIG. 26 show the efficiency of tandem U7-snRNAs targeting different target sites in inducing splicing skipping of exon 13 of USH2A pre-mRNA.

In this example, by applying tandem U7-snRNAs targeting different target sites, it was found that the efficiency of inducing splicing skipping of exon 13 of USH2A can be improved in reporter gene cells. In this example, there is no significant difference in the efficiency of inducing splicing skipping of exon 13 between U7 snRNA#24-#9 and U7 snRNA#9-#24, and there is no significant difference in the efficiency of inducing splicing skipping of exon 13 between U7 snRNA#25-#9 and U7 snRNA#9-#25, indicating that the U7 snRNA with tandem recognition domains has two or more recognition domains satisfying that each of the recognition domains is reverse complementary to its corresponding target site in the USH2A pre-mRNA, and there is no need to order the recognition domains from the 5' end to the 3' end of the U7 snRNA from the 3' end to the 5' end based on the positions of the target sites corresponding to the recognition domains in the USH2A pre-mRNA.

**Table 14: Efficiency of tandem U7-snRNAs targeting different target sites in inducing splicing skipping of exon 13 of USH2A pre-mRNA**

| Sample name | GFP-positive rate | | | Mean FITC intensity | | |
|---|---|---|---|---|---|---|
| | Round 1 | Round 2 | Round 3 | Round 1 | Round 2 | Round 3 |
| 293T | 0.04% | 0.02% | 0.03% | 7148 | 4472 | 4737 |
| Reporter group | 17.69% | 19.12% | 17.33% | 42200 | 44866 | 37795 |
| SC group | 25.85% | 20.16% | 23.64% | 62897 | 51724 | 56124 |
| snRNA#24-#9 | 83.97% | 88.02% | 87.11% | 2074370 | 2122694 | 1889718 |
| snRNA#25-#9 | 88.59% | 90.99% | 90.08% | 2335041 | 1844870 | 1539905 |
| snRNA#9-#24 | 85.97% | 86.9% | 85.08% | 2040456 | 1671371 | 1395412 |
| snRNA#9-#25 | 81.85% | 87.81% | 83.82% | 2001521 | 1927175 | 1565802 |

### Example 9: Induction of splicing skipping of exon 13 of USH2A by chemically synthesized U7 snRNA in WERI cells

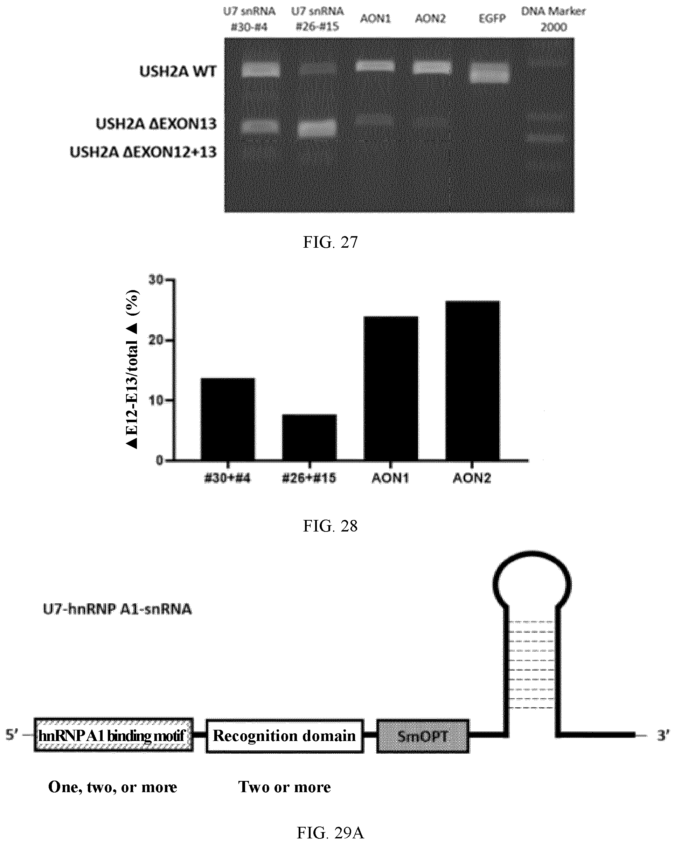

Human host cells were inoculated into a 24-well plate at a density of 6×10⁵ cells/well. In this example, the WERI-Rb-1 cell line was used. Lipofectamine 2000 was used to transfect WERI cells with 50 pmol of snRNA combination 1 (U7-snRNA#30 and U7-snRNA#4) and combination 2 (U7-snRNA#26 and U7-snRNA#15) synthesized *in vitro.* The same dose (50 pmol) of antisense oligonucleotides AON1 (5'-MA*MG*MC*MU*MU*MC*MG*MG*MA*MG*MA*MA*MA*MU*MU*MU*MA*MA *MA*MU*MC*-3'; "M" indicates 2'-O-methyl modification; "*" indicates phosphorothioate linkage; SEQ ID NO: 77) and AON2 (5'-MU*MG*MA*MU*MC*MA*MC*MA*MC*MC*MU*MA*MA*MG*MC*MC*MC*MU *MA*MA*MA*-3'; "M" indicates 2'-O-methyl modification; "*" indicates phosphorothioate linkage; SEQ ID NO: 78) were transfected as control groups. 1 µg of EGFP plasmid was transfected as a negative control. WERI cells without any plasmid transfection were used as a blank control. The transfected cells were cultured for 72 hours. RNA was then extracted from each experimental group and reverse transcribed to obtain cDNA, which was subjected to RT-PCR using primers AGCCTTTCCGCCAAGGTGATC (SEQ ID NO: 30) and CACAACGTTGCCCAGCAATGG (SEQ ID NO: 31) to detect the presence of exon splicing skipping in mature USH2A mRNA. The electrophoresis results are shown in FIG. 27. The RT-PCR electrophoresis bands were further quantitatively analyzed by ImageJ software, and the proportion of mature USH2A mRNAs with splicing skipping of exon 13 or splicing skipping of both exons 12 and 13 was statistically analyzed, as shown in FIG. 28.

In WERI cells endogenously expressing Usherin protein, the efficiency of U7 snRNA combinations targeting different target sites in inducing splicing skipping of exon 13 of USH2A pre-mRNA was compared with the preferred technical solutions of AONs in the prior art. It can be seen from the RT-PCR test data and analysis results that the efficiency of snRNA combination 1 and snRNA combination 2 in inducing splicing skipping of exon 13 was significantly superior to that of the optimal technical solutions of AON1 and AON2 in the prior art, and the proportion of mRNAs with double splicing skipping of exons 12 and 13 induced by snRNA combination 1 and snRNA combination 2 to the total mRNAs with splicing skipping was lower than that of AON1 and AON2. Thus, it is determined that U7 snRNA significantly improved the efficiency of single splicing skipping of exon 13 while ensuring a USH2A mRNA by-product with low double skipping.

Additionally, snRNA combination 2 had a target site adjacent to an AON site with a very high probability of inducing double splicing skipping of exons 12 and 13 in the prior art. However, the probability of double splicing skipping induced by snRNA combination 2 was very low.

### Example 10: Efficiency of splicing skipping of U7 snRNA with motifs that may recruit splicing regulatory proteins

Construction of U7 snRNA linked to hnRNP A1 binding motif. Corresponding Oligo DNAs were synthesized based on the pre-transcriptional DNA sequences corresponding to the sequences listed in the table, respectively. The sense strand of the Oligo DNA was the reverse complementary sequence of the target sequence (the DNA sequence corresponding to the recognition domain sequence), with CCGCAATATGATAGGGACTTAGGGTG (SEQ ID NO: 67) added at 5'. The antisense strand was the target sequence, with AATT added at 5' and CACCCTAAGTCCCTATCATATT (SEQ ID NO: 68) added at 3'. For example, if the recognition domain sequence was NNN (the recognition domain was preferably greater than 16 nucleotides in length), the sense strand of the synthesized Oligo DNA was CCGCAATATGA***TAGGGA***CT***TAGGGT***GNNN (SEQ ID NO: 81), and the antisense strand was AATTNNNC***ACCCTA***AG***TCCCTA***TCATATT (SEQ ID NO: 82) (the underlined portion indicates the double-stranded DNA sequence corresponding to the recognition domain sequence, and the bold italicized portion indicates the double-stranded DNA sequence corresponding to the binding motif "UAGGGU" or "UAGGGA" of hnRNP A1 protein).

The sense and antisense strands of the synthesized Oligo DNA were mixed according to an annealing reaction system (total reaction volume of 20 µL: 2 µL of Oligo-F (100 µM) + 2 µL of Oligo-R (100 µM) + 2 µL of 10×NEB Cutter smart buffer + 16 µL of deionized water). The mixture was incubated at 95°C for 5 minutes and then cooled on ice to form a double-stranded DNA with sticky ends. After 100-fold dilution, 1 µL of the double-stranded DNA was taken and ligated with 10 ng of linearized pUC57-U7 snRNA backbone plasmid recovered by BsaI digestion. The ligation product was further transformed into *E. coli* competent cells, then single clones were picked, followed by PCR and sequencing to obtain a U7 snRNA vector containing the hnRNP A1 binding motif for inducing splicing skipping of exon 13 of USH2A, named pUC57-U7-hnRNP A1-snRNA#A. The plasmid was purified and stored at -20°C for later use. FIG. 29A is a schematic diagram of the snRNA vector with hnRNP A1.

U7-hnRNP A1-snRNA can also be chemically synthesized and modified following the methods described in the examples of the present disclosure. Taking snRNA#15 and snRNA#25 as examples, the sequences and modifications of the chemically synthesized U7-hnRNP A1-snRNA are as follows (* indicates phosphorothioate backbone, m indicates 2'-O-methyl modification, the underlined portion indicates the recognition domain that is reverse complementary to the target sequence, the italicized portion indicates the smOPT sequence, and the bold portion indicates the binding motif of hnRNP A1 protein):

U7-hnRNP A1-snRNA#15:

5′-
mA*mC*mC*GCAAUAUGAUAGGGACUUAGGGUGACACUGGCAGGGCUCACAU
CCAAAUUUUUGGAGCAGGUUUCUGACUUCGGUCGGAAAACCmC*mC*mU*-3′
(SEQ ID NO: 79)

U7-hnRNP A1-snRNA#25:

5′-
mA*mC*mC*GCAAUAUGAUAGGGACUUAGGGUGAUUACACCUUCUUCCUUGA
CGAUUAAUUUUUGGAGCAGGUUUCUGACUUCGGUCGGAAACCmC*mC*mU*-
3′ (SEQ ID NO: 80)

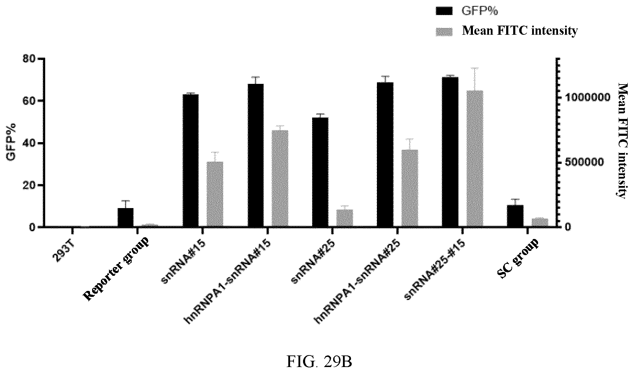

Induction of splicing skipping of exon 13 of USH2A in reporter gene cells by U7 snRNA linked to hnRNP A1 binding motif. 293T cells were inoculated into a 24-well plate at a density such that the confluence reached approximately 80% after 24 hours. Lipofectamine 2000 was used to co-transfect 293T cells with pCMV-EGFPleft-Exon13mut-EGFPright and pUC57-U7-hnRNP A1-snRNA#15 plasmid, pUC57-U7-hnRNP A1-snRNA#25 plasmid, pUC57-U7 snRNA#15 plasmid, pUC57-U7 snRNA#25 plasmid, and pUC57-U7 snRNA#25-#15 plasmid (with a vector mass ratio of 100 ng:400 ng), respectively. 293T cells transfected with the reporter plasmid alone (Reporter group) and 293T cells co-transfected with the reporter plasmid and pUC57-U7 Scramble (SC group) were used as two negative controls, while 293T cells without any plasmid transfection were used as a blank control. The transfected cells were cultured for 48 to 72 hours, trypsinized into single cells, and analyzed by flow cytometry to determine the efficiency of splicing skipping induced by different snRNA groups. Table 15 below and FIG. 29B show the efficiency of U7-hnRNP A1-snRNA in inducing splicing skipping of exon 13 of USH2A pre-mRNA.

The data showed that the introduction of the hnRNP A1 binding motif at the 5' end of U7 snRNA could significantly improve the efficiency of inducing splicing skipping of exon 13 of USH2A pre-mRNA, which not only increased the proportion of cells with splicing skipping of exon 13 (GFP+), but also increased the mRNA level of exon splicing skipping in each cell (mean FITC intensity). The efficiency of tandem snRNAs in inducing splicing skipping of exon 13, particularly the mean FITC intensity, was significantly superior to that of the introduction of the hnRNP A1 binding motif at the 5' end of U7 snRNA, suggesting that splicing skipping of exon 13 of USH2A pre-mRNA may be more sensitive to tandem snRNAs.

**Table 15: Efficiency of U7-hnRNP A1-snRNA in inducing splicing skipping of exon 13 of USH2A pre-mRNA**

| Sample name | GFP-positive rate | | | Mean FITC intensity | | |
|---|---|---|---|---|---|---|
| | Round 1 | Round 2 | Round 3 | Round 1 | Round 2 | Round 3 |
| 293T | 0.1% | 0.1% | 0.1% | 6366 | 5100 | 8240 |
| Reporter group | 9.2% | 12.8% | 6.1% | 23925 | 28917 | 17335 |
| snRNA#15 | 62.3% | 63.3% | 63.7% | 574041 | 428688 | 521495 |
| hnRNP A1-snRNA#15 | 66.3% | 71.8% | 66.6% | 783382 | 715282 | 754269 |
| snRNA#25 | 52.1% | 54.0% | 50.8% | 148755 | 110595 | 163122 |
| hnRNP A1-snRNA#25 | 67.9% | 72.1% | 66.3% | 680169 | 513447 | 603841 |
| snRNA#25-#15 | 70.8% | 71.9% | - | 1176740 | 929514 | - |
| SC group | 11.0% | 13.3% | 7.7% | 58920 | 75279 | 66826 |

In this example, a free tail was introduced at the 5' end of U7 snRNA. The free tail sequence comprises the binding motif "UAGGGU" or "UAGGGA" of hnRNP A1 protein and may contain one, two, or more, preferably two binding motifs of hnRNP A1 protein. The free tail sequence is preferably "UAUGAUAGGGACUUAGGGUG (SEQ ID NO: 35)", which can recruit hnRNP A1 proteins and promote splicing skipping of exon 13 of USH2A. Moreover, the structure is not applicable to snRNA with tandem recognition domains.

In some examples, the free tail introduced at the 5' end of U7 snRNA is a motif that can recruit splicing regulatory proteins. The splicing regulatory protein is hnRNP A1 (Heterogeneous Nuclear Ribonucleoprotein A1), SRSF1 (Serine and Arginine Rich Splicing Factor 1), RBM4 (RNA Binding Motif Protein 4), DAZAP1 (DAZ Associated Protein 1), SR (Serine and Arginine-Rich Protein), *etc.*

### Example 11: Construction of AAV-U7 snRNA-related plasmid vector and viral packaging for targeted induction of splicing skipping of exon 13 of USH2A pre-mRNA

In this example, the U7 snRNA gene for targeted induction of splicing skipping of exon 13 of USH2A pre-mRNA was inserted into and replaced the gene sequence between the two ITR domains of the pAAV-CMV vector to construct a pAAV-U7 snRNA vector. The pAAV-U7 snRNA vector was co-transfected into host cells with AAV packaging plasmids: serotype pRC plasmid (containing the AAV2 Rep gene and the respective Cap gene of each serotype) and pHelper plasmid (a vector plasmid containing the adenoviral E2A, E4, and VA genes) to package the AAV-U7 snRNA virus for targeted induction of splicing skipping of exon 13 of USH2A pre-mRNA. The specific operation process is as follows:

First, a gene sequence, *i.e.,* U7-snRNA gene expression cassette backbone (containing no recognition domain) was synthesized by means of total gene synthesis: 5'-mouse U7 promoter-smOPT sequence-U7 snRNA scaffold-snRNA gene-specific 3' cassette-3'. Two Type II restriction enzyme recognition sites (*e.g*., BsaI, AarI, BsmBI) were added between the U7 promoter and smOPT to facilitate subsequent excision, substitution, and insertion of other recognition domain sequences. The sequence synthesized by means of total gene synthesis was inserted into and replaced the gene sequence between the two AAV2-ITR domains of the pAAV-CMV plasmid (AAVpro^{®} Helper Free System (AAV5) kit, TAKARA Corporation, Code No. 6650) to obtain the pAAV-U7 snRNA backbone vector.

Following the method described in the above examples, the sense and antisense strands of corresponding Oligo DNAs were synthesized based on the pre-transcriptional DNA sequences corresponding to the snRNA recognition domain sequences or the tandem recognition domain sequences of the present disclosure, respectively, and both ends were added with sticky ends similar to those generated by the cleavage of Type II restriction enzyme recognition sites. The strands were annealed to form a double-stranded DNA with sticky ends (single/tandem recognition domain). The double-stranded DNA was ligated into the linearized pAAV-U7 snRNA backbone plasmid recovered by digestion with the corresponding Type II restriction enzyme using T4 ligase to form a pAAV-U7 snRNA plasmid targeting specific sites of exon 13 of USH2A pre-mRNA for inducing splicing skipping. The plasmid was named based on the corresponding snRNA ID of the recognition domain sequence, such as pAAV-U7 snRNA#25.

The target gene (U7-snRNA gene expression cassette for targeted induction of splicing skipping of exon 13 of USH2A pre-mRNA) was inserted into and replaced the gene sequence between the AAV2-ITR domains of the pAAV-CMV plasmid to obtain the pAAV-U7 snRNA plasmid vector. The AAV-U7 snRNA virus for targeted induction of splicing skipping of exon 13 of USH2A pre-mRNA was packaged according to the instructions of AAVpro^{®} Helper Free System (AAV5) kit and the standard cell operating procedures.

24 hours before transfection, HEK293/293T cells were inoculated into a 100 mm cell culture dish in a DMEM medium containing 10% FBS. Transfection was performed when the confluence reached 80% to 90%. 3 hours before transfection, the old medium was discarded and replaced with a fresh medium. During transfection, pAAV-U7 snRNA plasmid, pRC plasmid, pHelper plasmid, and PEI (polyethylenimine) transfection reagent were prepared according to the following system and added dropwise to the culture dish. After adding the PEI transfection mixture, the culture dish was gently shaken to ensure even distribution of the transfection reagent, and the medium was placed in a 37°C, 5% CO₂ incubator for incubation.

PEI transfection system: pAAV plasmid (1 µg/µL), 6 µL; pRC1/2/5/6 plasmids (1 µg/µL); (pRC plasmid capsid gene determines serotype), 6 µL; pHelper plasmid (1 µg/µL), 6 µL; serum-free DMEM medium, 500 µL; PEI (1 mg/mL), 110 µL. Treatment: vortex several times and incubate at room temperature for 5 minutes.

24 hours after transfection, the medium was replaced with a fresh DMEM medium containing 2% FBS. 48 to 72 hours after transfection, cells containing AAV virus were harvested, washed, and centrifuged to obtain cell pellets, which were collected and vortexed to loosen. Subsequently, according to the instructions of AAVpro^{®} Helper Free System (AAV5) kit, 0.5 mL of AAV Extraction Solution A was added to the cell pellet, and the mixture was vortexed for 15 seconds to fully suspend the cell pellet. After standing for 5 minutes at room temperature, the mixture was vortexed for another 15 seconds. Cell debris was removed by centrifugation at 2000 to 14000 g for 10 minutes at 4°C. The supernatant was collected into a new sterile centrifuge tube, and 50 µL of AAV Extraction Solution B was added. The resulting mixture was mixed well using a pipette to obtain an AAV-U7 snRNA virus solution with different recognition domains. A portion of the virus solution was taken to measure the viral titer using qPCR, and the remaining solution was stored at 80°C for later use.

Since the target gene fragment inserted between the AAV2-ITR domains of the pAAV-U7 snRNA plasmid should be less than 2.5 kb, a plurality of U7-snRNA gene expression cassettes (5'-mouse U7 promoter-smOPT sequence, U7 snRNA scaffold-snRNA gene-specific 3' cassette-3') can be inserted to ensure increased expression of U7 snRNA under the condition of the same number of AAV particles. Given that the gene sequence is approximately 450 bp in length, the pAAV-U7 snRNA plasmid preferably carries 1 to 5 U7-snRNA gene expression cassettes. The plurality of U7-snRNA gene expression cassettes in the pAAV-U7 snRNA plasmid may have the same recognition domain or a combination of recognition domains, or may have a combination of different or not identical recognition domains.

In the present disclosure, U7 snRNA delivered by AAV induces splicing skipping of exon 13 of USH2A pre-mRNA. The capsid protein of the AAV may be a naturally-derived capsid protein or a mutant of the naturally-derived capsid protein, or undergo directed evolution or rational modification of amino acids/peptides (codon optimization, chimerization of functional peptides with different serotypes, *etc*.) to improve the characteristics such as tissue tropism, immunogenicity, and transfection efficiency, such as AAV2.5, AAV2i8, AAV-TT, AAV9.HR, and CAM130.

### Example 12: U7 snRNA tandem significantly outperforms U7 snRNA combination in inducing splicing skipping - in vitro dose escalation

Following the method described in Example 11, the following vectors of AAV2 serotype were constructed: AAV2-3×U7 snRNA#9-#25 (3×U7 snRNA tandem), AAV2-2×U7 snRNA#9-2×U7 snRNA#25 (4×U7 snRNA separate), and AAV2-2×U7-hnRNP A1-snRNA#9-2×U7-hnRNP A1-snRNA#25 (4×U7 snRNA separate-motif). These vectors were transfected into HEK293/293T cells for viral packaging. After collection and purification, the following AAV2 viruses were obtained: 3×U7 snRNA tandem, 4×U7 snRNA separate, and 4×U7 snRNA separate-motif.

The viral titer was measured, and the corresponding volume of virus solution was added to WERI-Rb-1 cells in a 24-well plate (6×10⁵ cells/well) at MOI values of 3×10⁵, 1×10⁵ 3×10⁴, 1×10⁴, 3×10³, 1×10³, and 3×10² (MOI value = viral titer (TU/mL) × virus volume (mL) / number of cells), respectively. After infection, the cells were cultured for 72 hours. RNA was then extracted from each experimental group and reverse transcribed to obtain cDNA, which was subjected to RT-PCR and qRT-PCR using the corresponding primers/probes listed in Table 16 to detect the efficiency of AAV-U7 snRNA in inducing splicing skipping of exon 13 of USH2A pre-mRNA.

**Table 16: List of RT-PCR primers and qRT-PCR probes**

| Purpose | Primer/probe name | Primer/probe sequences | SEQ ID |
|---|---|---|---|
| RT-PCR detection of wild-type USHER mRNA/USHER mRNA with splicing skipping of exon 13 | USH2A-RTPCR-F1 | AGCCTTTCCGCCAAGGTGATC | 30 |
| | USH2A-RTPCR-R1 | CACAACGTTGCCCAGCAATGG | 31 |
| qRT-PCR detection of wild-type USH2A mRNA | WT-probe-QPCR-F | GGTTAGATGTCACCAATTGTAAGG | 69 |
| | WT-probe-QPCR-R | AACATTGGGCTTGCAGATGCAC | 70 |
| | WT-probe-QPCR-P | 5FAM-CTGTCTTAGCATTACAG-3MGB | 71 |
| qRT-PCR detection of USH2A mRNA with splicing skipping of exon 13 | dEX13-probe-QPCR-F | GATGGCTGCAGTCCCTGTAAC | 72 |
| | dEX13-probe-QPCR-R | GCCTGGAGAAATATAAAAACCAATAAC | 73 |
| | dEX13-probe-QPCR-P | 5FAM-CAATACCTCTGGGACA-3MGB | 74 |

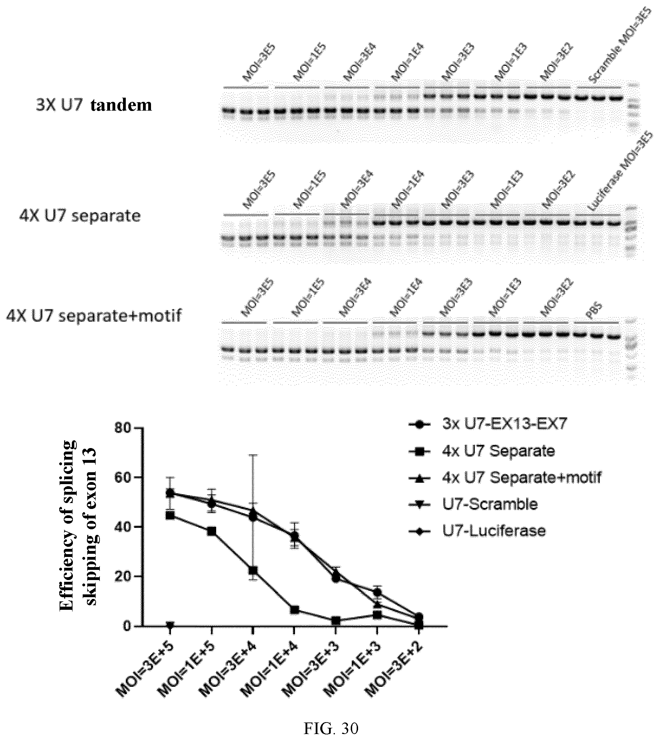

FIG. 30 shows a comparison of 3×U7 tandem and 4×U7 separate by AAV2 viral transduction. The RT-PCR results showed that the efficiency of 4×U7 separate was poor, but the efficiency was improved by the addition of the hnRNP A1 binding motif, which was comparable to the efficiency of 3×U7 tandem. The qRT-PCR results were consistent with the RT-PCR results.

Since the maximum capacity of the ssAAV vector for the target gene is 4.7 kb, the maximum capacity of the scAAV vector for the target gene is 2.5 kb, and the size of the U7 snRNA expression cassette is approximately 450 bp, a single scAAV vector can accommodate up to 9 U7 snRNA expression cassettes, and a single ssAAV vector can accommodate up to 5 U7 snRNA expression cassettes. Based on the results of Example 14, the 3×U7 snRNA tandem showed similar efficiency to the 2×U7 snRNA-hnRNP A1 combination in inducing splicing skipping *in vitro* and outperformed the 2×U7 snRNA combination. Based on the results of Examples 15 and 16, the 1×U7 snRNA tandem showed slightly better efficiency than the 2×U7 snRNA combination in inducing splicing skipping *in vivo,* but with more significant long-term persistence. Without the introduction of the hnRNP A1 binding motif, the U7 snRNA tandem outperformed the U7 snRNA combination.

### Example 13: AAV-U7 snRNA significantly outperforms AON in inducing splicing skipping - in vitro dose escalation

Following the method described in Example 11, AAV2-U7 snRNA#9-#25 (1×U7 snRNA tandem), a vector of AAV2 serotype, was constructed. The vector was transfected into HEK293/293T cells for viral packaging. After collection and purification, the AAV2 virus containing 1×U7 snRNA tandem, *i.e.,* AAV2-RM-101, was obtained. The viral titer was measured and the virus was stored for later use.

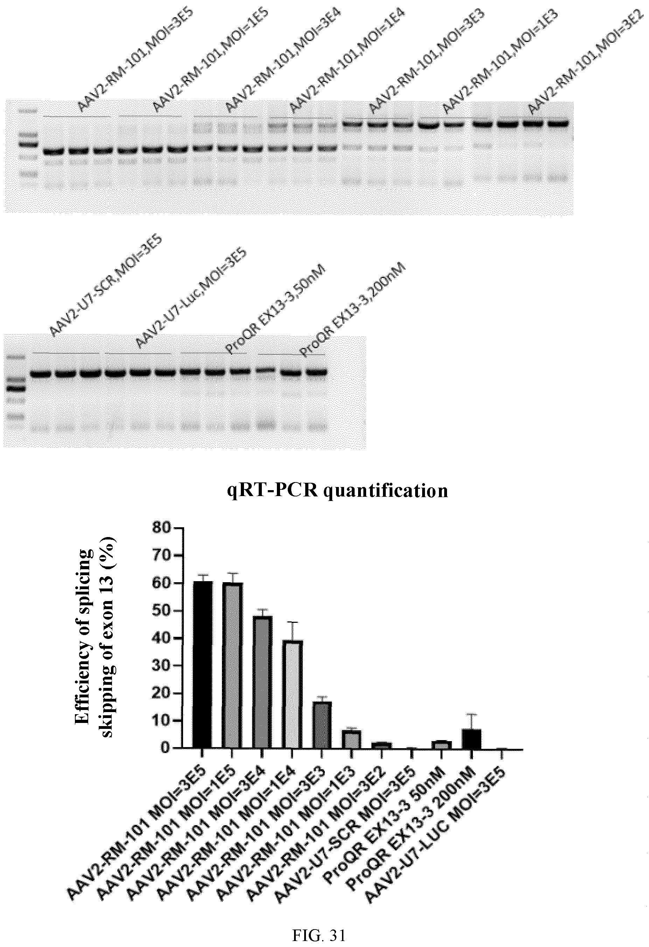

WERI-Rb-1 cells were inoculated into a 24-well plate at a density of 6×10⁵ cells/well. The AAV2-RM-101 virus was added to the cells at MOI values of 3×10⁵, 1×10⁵, 3×10⁴, 1×10⁴, 3×10³, 1×10³, and 3×10², respectively, serving as experimental groups with different MOI values. PROQR EX13-3 (AON1 in Example 9) at 50 nM and 200 nM were used as positive controls. AAV2-U7-SCR (Scramble) and AAV2-U7-LUC (Luciferase recognition) at an MOI value of 3×10⁵ were used as negative controls. After treatments, the WERI-Rb-1 cells were cultured for 72 hours. RNA was then extracted from each experimental group and reverse transcribed to obtain cDNA, which was subjected to RT-PCR and qRT-PCR using the corresponding primers/probes listed in Table 16 to detect the efficiency of splicing skipping of exon 13 of USH2A pre-mRNA. As shown in FIG. 31, the results demonstrated that AAV2-U7 snRNA#9-#25 significantly outperformed AON1 in inducing splicing skipping.

### Example 14: Induction of splicing skipping of exon 13 of USH2A pre-mRNA in humanized mouse retinal cells by snRNA

Exon 12 and partial flanking sequence [approximately 1670 bp upstream of exon 12 (3' end of mouse intron 11) to approximately 1600 bp downstream of exon 12 (5' end of mouse intron 12)] of the USH2A gene in C57/BL6J mice were replaced with exon 13 and partial flanking sequence [approximately 1611 bp upstream of exon 13 (3' end of human intron 12) to approximately 1599 bp downstream of exon 13 (5' end of human intron 13)] of the human USH2A gene as well as insertion sequence using gene editing technology. Additionally, c.2208T to G was introduced into exon 13 of human USH2A, resulting in humanized mice carrying the c.2802T>G mutation in exon 13 of USH2A (USH2A EXON13^{c.2802T>G}).

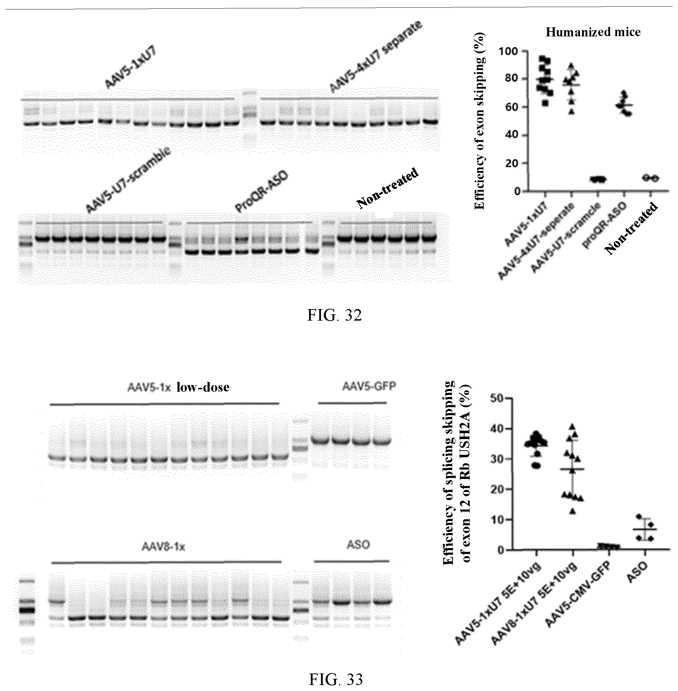

1 µL of AAV2-U7 snRNA#9-#25 (AAV5-1×U7) and AAV5-2×U7 snRNA#9-2×U7 snRNA#25 (AAV5-4×U7 separate) viruses were injected into the eyes of hUSH2A EXON13^{c.2802T>G} knock-in humanized mice at a dose of 1E+10 vg/eye by subretinal injection. Mice injected with AAV5-U7-scramble virus served as the negative control group. Mice intravitreally injected with PROQR-AON (AON1 in Example 9) at a dose of 15 µg/eye (1 µL) served as the positive control group. Untreated mice served as the blank group (non-treated group). Both eyes of each mouse were injected. Three weeks post-injection, the experimental mice were euthanized, and retinal tissues were collected. RNA was then extracted and reverse transcribed to obtain cDNA, which was subjected to RT-PCR and qRT-PCR using the corresponding primers/probes listed in Table 16 to detect the efficiency of splicing skipping of exon 13 of USH2A pre-mRNA. As shown in FIG. 32, the results demonstrated that 1×U7 snRNA tandem showed better efficiency in inducing splicing skipping in the retinal tissues compared to the 2×U7 snRNA combination, and both outperformed PROQR AON (AON1 in Example 9) (according to the results from the non-treated group, exon 13 of human USH2A containing the mutation exhibited a certain degree of spontaneous skipping, which was consistent with existing research reports).

### Example 15: Induction of splicing skipping of exon 12 of USH2A pre-mRNA in rabbit eye cells by injection of AAV-U7 snRNA of different serotypes

AAV5-U7 snRNA#9-#25 (AAV5-1×U7) at MOI values of 5×10¹⁰ and 2×10¹¹ and AAV8-U7 snRNA#9-#25 (AAV8-1×U7) at an MOI value of 5×10¹⁰ were injected into the eyes of rabbits by subretinal injection. Rabbits subretinally injected with AAV5-CMV-GFP virus served as the negative control group. Rabbits intravitreally injected with AON (AON1 in Example 9) at a dose of 50 µg (50 µL) served as the positive control group. Two weeks post-injection, the experimental rabbits were euthanized, and retinal tissues were collected. RNA was then extracted and reverse transcribed to obtain cDNA, which was subjected to RT-PCR and qRT-PCR using the corresponding primers/probes listed in Table 16 to detect the efficiency of splicing skipping of exon 13 of USH2A pre-mRNA.

As shown in FIG. 33, the results demonstrated that U7 snRNA delivered by AAV5 showed better efficiency in inducing splicing skipping compared to AAV8, and U7 snRNA tandem delivered by different AAV serotypes outperformed AON.

The USH2A gene in rabbits is wild-type rabbit USH2A without a mutation in exon 12 (equivalent to exon 13 of human USH2A). In contrast, in hUSH2A EXON13^{c.2802T>G} knock-in humanized mice, human exon 13 containing the mutation is more susceptible to induced splicing skipping. The efficiency of AAV-U7 snRNA and AON in inducing splicing skipping of target exons in rabbits was significantly lower than that in humanized mice, suggesting that the efficiency of AON is more easily influenced by exon sequences and mutations.

### Example 16: Long-term effects of U7 snRNA delivered by AAV in inducing splicing skipping of exon 13 of USH2A pre-mRNA

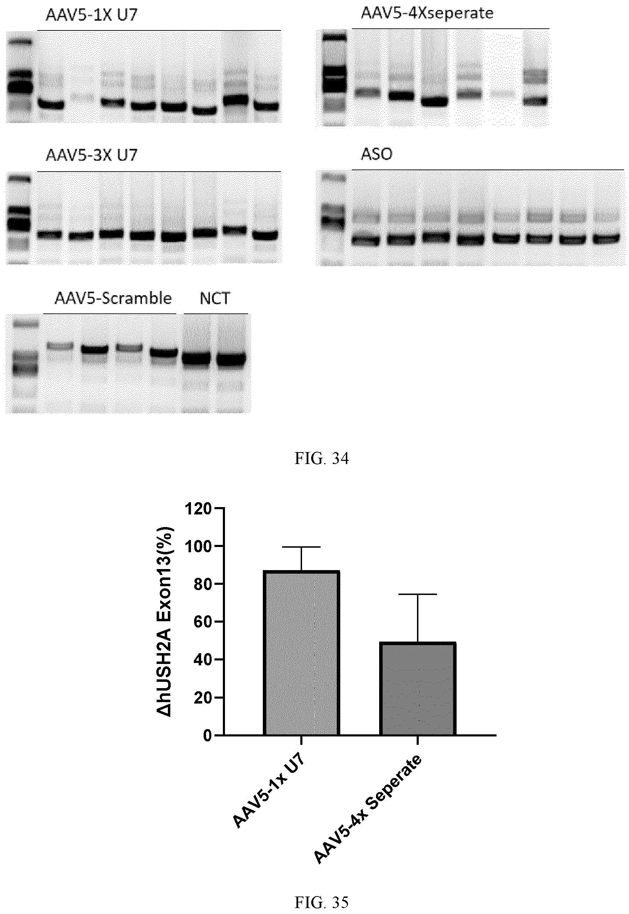

AAV5-3×U7 snRNA#9-#25 (AAV5-3 ×U7), AAV5-U7 snRNA#9-#25 (AAV5-1×U7), and AAV5-2×U7 snRNA#9-2×U7 snRNA#25 (AAV5-4×U7 separate) viruses were injected into the eyes of hUSH2A EXON13^{c.2802T>G} knock-in humanized mice at a dose of 1E+10 vg/eye (1 µL) by subretinal injection. Mice injected with AAV5-U7-scramble virus served as the negative control group. Mice intravitreally injected with PROQR-ASO (AON1 in Example 9) at a dose of 15 µg/eye (1 µL) served as the positive control group. Untreated mice served as the blank group (NTC). Twenty-two weeks post-injection, the experimental mice were euthanized, and retinal tissues were collected. RNA was then extracted and reverse transcribed to obtain cDNA, which was subjected to RT-PCR using the corresponding primers listed in Table 7 to detect the efficiency of splicing skipping of exon 13 of USH2A pre-mRNA. The results (FIGs. 34 to 35) showed that, 22 weeks later, U7 snRNA tandem delivered by AAV still exhibited the optimal efficiency of splicing skipping with long-term maintenance. In this example, to further compare the long-term maintenance between U7 snRNA tandem and U7 snRNA combination, qRT-PCR was further performed using the probes listed in Table 16 to detect and compare the differences in long-term maintenance of efficiency of splicing skipping of exon 13 of USH2A pre-mRNA between AAV5-1 ×U7 and AAV5-4×U7 separate in the retina. The results (FIG. 34) showed that, 22 weeks later, U7 snRNA tandem still exhibited the optimal efficiency of splicing skipping, with a potential cumulative therapeutic effect compared to the 3-week results. Moreover, the persistence of AAV-1×U7 snRNA tandem was significantly superior to that of AAV-4×U7 separate.

## Claims

1. An snRNA nucleic acid molecule, comprising: a recognition domain, a stem-loop sequence, and an Sm sequence; wherein the number of the recognition domains is at least two;
wherein each of the recognition domains from the 5' end to the 3' end is reverse complementary to a target sequence fragment from the 3' end to the 5' end of a pre-mRNA;
the pre-mRNA is a pre-mRNA corresponding to the USH2A gene.

2. The snRNA nucleic acid molecule according to claim 1, wherein each of the recognition domains from the 5' end to the 3' end is sequentially reverse complementary to the target sequence fragment from the 3' end to the 5' end of the pre-mRNA.

3. The snRNA nucleic acid molecule according to claim 1, wherein each of the recognition domains from the 5' end to the 3' end is non-sequentially reverse complementary to the target sequence fragment from the 3' end to the 5' end of the pre-mRNA.

4. The snRNA nucleic acid molecule according to any one of claims 1 to 3, wherein the recognition domain has a length of at least 16 bp.

5. The snRNA nucleic acid molecule according to any one of claims 1 to 4, wherein the recognition domain has a length of 18 to 40 bp.

6. The snRNA nucleic acid molecule according to any one of claims 1 to 5, wherein the recognition domain has a length of 20 to 27 bp.

7. The snRNA nucleic acid molecule according to any one of claims 1 to 6, wherein the number of the recognition domains is two.

8. The snRNA nucleic acid molecule according to claim 7, wherein the snRNA nucleic acid molecule comprises, sequentially from the 5' end to the 3' end: two adjacent recognition domains, an Sm sequence, and a stem-loop sequence.

9. The snRNA nucleic acid molecule according to any one of claims 1 to 8, wherein the pre-mRNAis all or part of the pre-mRNA corresponding to intron 12 to intron 13 of the USH2A gene.

10. The snRNA nucleic acid molecule according to claim 9, wherein the pre-mRNA is all or part of the pre-mRNA corresponding to exon 13 of the USH2A gene.

11. The snRNA nucleic acid molecule according to claim 9, wherein the genomic location of the pre-mRNA is Chr1: 216246563-216247246; the target sequence fragment is selected from the nucleotide sequence as shown in SEQ ID NO: 1 and a variant sequence thereof.

12. The snRNA nucleic acid molecule according to claim 11, wherein the genomic location of the pre-mRNA is Chr1: 216246563-216246753; the target sequence fragment is selected from the nucleotide sequence as shown in SEQ ID NO: 3 and a variant sequence thereof.

13. The snRNA nucleic acid molecule according to claim 12, wherein the genomic location of the pre-mRNA is Chr1: 216246563-216246649; the target sequence fragment is selected from the nucleotide sequence as shown in SEQ ID NO: 4 and a variant sequence thereof.

14. The snRNA nucleic acid molecule according to claim 13, wherein the genomic location of the pre-mRNA is Chr1: 216246563-216246626; the target sequence fragment is selected from the nucleotide sequence as shown in SEQ ID NO: 9 and a variant sequence thereof; or
the genomic location of the pre-mRNA is Chr1: 216246616-216246649; the target sequence fragment is selected from the nucleotide sequence as shown in SEQ ID NO: 34 and a variant sequence thereof.

15. The snRNA nucleic acid molecule according to claim 11, wherein the genomic location of the pre-mRNA is Chr1: 216247130-216247246; the target sequence fragment is selected from the nucleotide sequence as shown in SEQ ID NO: 2 and a variant sequence thereof.

16. The snRNA nucleic acid molecule according to claim 15, wherein the genomic location of the pre-mRNA is Chr1: 216247142-216247185; the target sequence fragment is selected from the nucleotide sequence as shown in SEQ ID NO: 32 and a variant sequence thereof; or
the genomic location of the pre-mRNA is Chr1: 216247130-216247161; the target sequence fragment is selected from the nucleotide sequence as shown in SEQ ID NO: 33 and a variant sequence thereof; or
the genomic location of the pre-mRNA is Chr1: 216247210-216247246; the target sequence fragment is selected from the nucleotide sequence as shown in SEQ ID NO: 36 and a variant sequence thereof; or
the genomic location of the pre-mRNA is Chr1: 216247204-216247232; the target sequence fragment is selected from the nucleotide sequence as shown in SEQ ID NO: 37 and a variant sequence thereof; or
the genomic location of the pre-mRNA is Chr1: 216247187-216247220; the target sequence fragment is selected from the nucleotide sequence as shown in SEQ ID NO: 38 and a variant sequence thereof; or
the genomic location of the pre-mRNA is Chr1: 216247169-216247202; the target sequence fragment is selected from the nucleotide sequence as shown in SEQ ID NO: 39 and a variant sequence thereof.

17. The snRNA nucleic acid molecule according to any one of claims 1 to 16, wherein the number of the recognition domains is two, and the two recognition domains are adjacent to each other.

18. The snRNA nucleic acid molecule according to claim 17, wherein the two recognition domains are, from the 5' end to the 3' end, a first recognition domain and a second recognition domain, respectively;
wherein the target sequence fragment reverse complementary to the first recognition domain or the second recognition domain is selected from the nucleotide sequence as shown in SEQ ID NO: 34 and a variant sequence thereof and the nucleotide sequence as shown in SEQ ID NO: 9 and a variant sequence thereof; correspondingly, the target sequence fragment reverse complementary to the second recognition domain or the first recognition domain is selected from the nucleotide sequence as shown in SEQ ID NO: 32 and a variant sequence thereof, the nucleotide sequence as shown in SEQ ID NO: 33 and a variant sequence thereof, the nucleotide sequence as shown in SEQ ID NO: 36 and a variant sequence thereof, the nucleotide sequence as shown in SEQ ID NO: 37 and a variant sequence thereof, the nucleotide sequence as shown in SEQ ID NO: 38 and a variant sequence thereof, and the nucleotide sequence as shown in SEQ ID NO: 39 and a variant sequence thereof.

19. The snRNA nucleic acid molecule according to claim 18, wherein the target sequence fragment reverse complementary to the first recognition domain is selected from the nucleotide sequence as shown in SEQ ID NO: 34 and a variant sequence thereof and the nucleotide sequence as shown in SEQ ID NO: 9 and a variant sequence thereof; the target sequence fragment reverse complementary to the second recognition domain is selected from the nucleotide sequence as shown in SEQ ID NO: 32 and a variant sequence thereof, the nucleotide sequence as shown in SEQ ID NO: 33 and a variant sequence thereof, the nucleotide sequence as shown in SEQ ID NO: 36 and a variant sequence thereof, the nucleotide sequence as shown in SEQ ID NO: 37 and a variant sequence thereof, the nucleotide sequence as shown in SEQ ID NO: 38 and a variant sequence thereof, and the nucleotide sequence as shown in SEQ ID NO: 39 and a variant sequence thereof.

20. The snRNA nucleic acid molecule according to claim 19, wherein the nucleotide sequence of the first recognition domain is as shown in any one of SEQ ID NOs: 12 to 22 and 59 to 61, and the nucleotide sequence of the second recognition domain is as shown in any one of SEQ ID NOs: 40 to 58.

21. The snRNA nucleic acid molecule according to claim 20, wherein the nucleotide sequence of the first recognition domain is as shown in SEQ ID NO: 12, 13, 15, or 17, and the nucleotide sequence of the second recognition domain is as shown in SEQ ID NO: 48, 54, 56, or 58.

22. The snRNA nucleic acid molecule according to claim 21, wherein the nucleotide sequence of the first recognition domain is as shown in SEQ ID NO: 12, 13, or 17, and the nucleotide sequence of the second recognition domain is as shown in SEQ ID NO: 48, 54, or 58.

23. The snRNA nucleic acid molecule according to claim 20, wherein the nucleotide sequence of the first recognition domain is as shown in SEQ ID NO: 12, and the nucleotide sequence of the second recognition domain is as shown in SEQ ID NO: 48; or
the nucleotide sequence of the first recognition domain is as shown in SEQ ID NO: 12, and the nucleotide sequence of the second recognition domain is as shown in SEQ ID NO: 54; or
the nucleotide sequence of the first recognition domain is as shown in SEQ ID NO: 12, and the nucleotide sequence of the second recognition domain is as shown in SEQ ID NO: 58; or
the nucleotide sequence of the first recognition domain is as shown in SEQ ID NO: 13, and the nucleotide sequence of the second recognition domain is as shown in SEQ ID NO: 48; or
the nucleotide sequence of the first recognition domain is as shown in SEQ ID NO: 13, and the nucleotide sequence of the second recognition domain is as shown in SEQ ID NO: 54; or
the nucleotide sequence of the first recognition domain is as shown in SEQ ID NO: 13, and the nucleotide sequence of the second recognition domain is as shown in SEQ ID NO: 58; or
the nucleotide sequence of the first recognition domain is as shown in SEQ ID NO: 17, and the nucleotide sequence of the second recognition domain is as shown in SEQ ID NO: 48; or
the nucleotide sequence of the first recognition domain is as shown in SEQ ID NO: 17, and the nucleotide sequence of the second recognition domain is as shown in SEQ ID NO: 54; or
the nucleotide sequence of the first recognition domain is as shown in SEQ ID NO: 17, and the nucleotide sequence of the second recognition domain is as shown in SEQ ID NO: 58; or
the nucleotide sequence of the first recognition domain is as shown in SEQ ID NO: 16, and the nucleotide sequence of the second recognition domain is as shown in SEQ ID NO: 42; or
the nucleotide sequence of the first recognition domain is as shown in SEQ ID NO: 18, and the nucleotide sequence of the second recognition domain is as shown in SEQ ID NO: 43; or
the nucleotide sequence of the first recognition domain is as shown in SEQ ID NO: 14, and the nucleotide sequence of the second recognition domain is as shown in SEQ ID NO: 55.

24. The snRNA nucleic acid molecule according to any one of claims 11 to 23, wherein the variant sequence is the presence of a substitution, addition, or deletion of one or more nucleotides in the nucleotide sequence.

25. The snRNA nucleic acid molecule according to claim 24, wherein the variant sequence is the presence of a substitution of one or more nucleotides in the nucleotide sequence.

26. The snRNA nucleic acid molecule according to claim 24, wherein the mutation is selected from a natural pathogenic mutation and a natural non-pathogenic mutation; wherein the natural pathogenic mutation is one or more selected from c.2242C>T, c.2276G>T, c.2299delG, c.2522C>A, c.2541C>A, c.2761delC, c.2776C>T, c.2802T>G, c.2209C>T, c.2310delA, c.2391_2392deITG, c.2431A>T, c.2431_2432delAA, c.2440C>T, c.2525dup, c.2610C>A, c.2755C>T, c.2176T>C, c.2236C>G, c.2296T>C, and c.2332G>T.

27. The snRNA nucleic acid molecule according to claim 26, wherein the natural pathogenic mutation is one or more selected from c.2802T>G, c.2299delG, and c.2276G>T.

28. The snRNA nucleic acid molecule according to claim 27, wherein the natural pathogenic mutation is c.2802T>G.

29. The snRNA nucleic acid molecule according to any one of claims 1 to 28, wherein the Sm sequence is a consensus sequence, and the stem-loop sequence comprises a stem-loop sequence of U1, U2, U3, U4, U5, U6, or U7.

30. The snRNA nucleic acid molecule according to claim 29, wherein the stem-loop sequence is a stem-loop sequence of U7.

31. The snRNA nucleic acid molecule according to claim 29, wherein the stem-loop sequence is a stem-loop sequence of U1.

32. The snRNA nucleic acid molecule according to claim 29, wherein the number of the stem-loop sequences is 1 to 2.

33. The snRNA nucleic acid molecule according to claim 29, wherein the Sm sequence is as shown in SEQ ID NO: 6.

34. The snRNA nucleic acid molecule according to claim 29, wherein the stem-loop sequence is as shown in SEQ ID NO: 7.

35. The snRNA nucleic acid molecule according to any one of claims 1 to 34, wherein the snRNA nucleic acid molecule comprises a modified nucleotide or an analog monomer thereof.

36. The snRNA nucleic acid molecule according to claim 35, further comprising a unidirectional extension sequence or a bidirectional extension sequence on the nucleotides at the 5' end and/or the 3' end of the recognition domain.

37. The snRNA nucleic acid molecule according to claim 35, wherein the modification is selected from a 2'-O-alkyl modification, a 2'-O-methoxy modification, and a 2'-O-methoxyethyl modification; wherein the 2'-O-alkyl modification is preferably a 2'-O-methyl modification.

38. The snRNAnucleic acid molecule according to claim 37, wherein the analog monomer is selected from a 6'-modified bicyclic nucleoside, a 5'-modified bicyclic nucleoside, a 6'-disubstituted bicyclic nucleoside, a tetrahydropyran nucleoside analog, and a 2'-deoxy-2'-fluoro-β-D-arabinonucleotide.

39. The snRNA nucleic acid molecule according to any one of claims 1 to 38, wherein the nucleotides of the snRNA nucleic acid molecule are linked by a chemical bond, and the chemical bond is selected from a phosphate bond, a methylene bond, an amide bond, a methylphosphonate bond, and a 3'-thioacetal bond.

40. The snRNA nucleic acid molecule according to claim 39, wherein the phosphate bond is selected from a phosphorothioate bond, a dithiophosphate bond, an alkylphosphonate bond, a phosphoroamidate bond, a boranophosphate bond, and a chiral linkage phosphorus.

41. The snRNA nucleic acid molecule according to claim 40, wherein the phosphate bond is selected from a phosphorothioate bond.

42. The snRNA nucleic acid molecule according to any one of claims 35 to 41, wherein the snRNA nucleic acid molecule comprises a modified nucleotide or an analog monomer thereof at positions 1 to 80 from the 5' end and/or the 3' end.

43. The snRNA nucleic acid molecule according to claim 42, wherein the snRNA nucleic acid molecule comprises a modified nucleotide or an analog monomer thereof at positions 3 to 40 from the 5' end and/or the 3' end.

44. The snRNA nucleic acid molecule according to claim 43, wherein the snRNA nucleic acid molecule comprises a modified nucleotide or an analog monomer thereof at positions 6 to 10 from the 5' end and/or the 3' end.

45. The snRNA nucleic acid molecule according to any one of claims 35 to 44, wherein the snRNA nucleic acid molecule comprises at least one phosphate bond from the 5' end or the 3' end.

46. The snRNA nucleic acid molecule according to claim 45, wherein the snRNA nucleic acid molecule comprises 1 to 3 phosphate bonds from the 5' end; or
the snRNA nucleic acid molecule comprises 1 to 3 phosphate bonds from the 3' end.

47. A combination of snRNA nucleic acid molecules, comprising one or more snRNA nucleic acid molecules according to any one of claims 1 to 46.

48. The combination according to claim 47, wherein at least two recognition domains are located on the same or different snRNA nucleic acid molecules.

49. A DNA molecule encoding the snRNA nucleic acid molecule according to any one of claims 1 to 46 or the combination according to claim 47 or 48.

50. A gene expression cassette, comprising a promoter and the DNA molecule according to claim 49.

51. The gene expression cassette according to claim 50, wherein a cleavable site, such as a Type II restriction enzyme recognition site, is further comprised between the promoter and the Sm sequence of the DNA molecule.

52. The gene expression cassette according to claim 50 or 51, wherein the promoter is a U7 promoter.

53. The gene expression cassette according to claim 52, wherein the promoter is a murine U7 promoter.

54. The gene expression cassette according to any one of claims 50 to 53, wherein the gene expression cassette comprises a recognition domain and a scaffold sequence; the scaffold sequence is as shown in SEQ ID NO: 62.

55. A recombinant expression vector, comprising the snRNA nucleic acid molecule according to any one of claims 1 to 46, the combination according to claim 47 or 48, or the gene expression cassette according to any one of claims 50 to 54.

56. The recombinant expression vector according to claim 55, wherein the recombinant expression vector is an expression vector selected from a plasmid, a phage, a minicircle DNA, a linear DNA, and a virus.

57. The recombinant expression vector according to claim 56, wherein the expression vector is a lentivirus or an adeno-associated virus.

58. The recombinant expression vector according to claim 57, wherein the capsid protein of the adeno-associated virus is a naturally-derived capsid protein or a mutant thereof, and the plasmid of the adeno-associated virus is a single-stranded AAV or a self-complementary AAV.

59. The recombinant expression vector according to claim 58, wherein the naturally-derived capsid protein is selected from AAV1, AAV2, AAV3, AAV4, AAV5, AAV6, AAV7, AAV8, AAV9, AAVrh.8, AAVrh.10, and AAVrh.43;
the mutant is selected from AAV2.5, AAV2i8, AAV-TT, AAV9.HR, and CAM130.

60. A virus particle, comprising a capsid protein and a nucleic acid, wherein the nucleic acid comprises the snRNA nucleic acid molecule according to any one of claims 1 to 46, the combination according to claim 47 or 48, or the DNA molecule according to claim 49.

61. The virus particle according to claim 60, wherein the capsid protein is a capsid protein derived from an adeno-associated virus.

62. The virus particle according to claim 61, wherein the capsid protein derived from the adeno-associated virus is a naturally-derived capsid protein or a mutant thereof.

63. The virus particle according to claim 62, wherein the naturally-derived capsid protein is selected from AAV1, AAV2, AAV3, AAV4, AAV5, AAV6, AAV7, AAV8, AAV9, AAVrh.8, AAVrh.10, and AAVrh.43; the mutant is selected from AAV2.5, AAV2i8, AAV-TT, AAV9.HR, and CAM130.

64. A pharmaceutical composition, comprising the snRNA nucleic acid molecule according to any one of claims 1 to 46, the combination according to claim 47 or 48, the DNA molecule according to claim 49, the gene expression cassette according to any one of claims 50 to 54, the recombinant expression vector according to claims 55 to 59, or the virus particle according to any one of claims 60 to 63.

65. The pharmaceutical composition according to claim 64, further comprising a pharmaceutically acceptable carrier.

66. A method for inducing the production of an Usherin protein lacking exon 13, comprising introducing into a host cell the snRNA nucleic acid molecule according to any one of claims 1 to 46, the combination according to claim 47 or 48, the DNA molecule according to claim 49, the gene expression cassette according to any one of claims 50 to 54, the recombinant expression vector according to claims 55 to 59, the virus particle according to any one of claims 60 to 63, or the pharmaceutical composition according to claim 64 or 65, thereby causing splicing skipping of exon 13.

67. The method according to claim 66, wherein the host cell is selected from retinal tissue cells, inner ear cells, cells with differentiation potential into retinal tissue cells and/or inner ear cells, and cells capable of performing functions corresponding to retinal tissue cells and/or inner ear cells.

68. The method according to claim 67, wherein the retinal tissue cells are retinal photoreceptor cells, and the inner ear cells are inner ear hair cells.

69. The method according to claim 67, wherein the cells with differentiation potential are selected from induced pluripotent stem cells, embryonic stem cells, neural precursor cells, retinal progenitor cells, retinal precursor cells, and mesenchymal stromal cells.

70. A method for inhibiting the expression and/or function of exon 13 of USH2A pre-mRNA, comprising administering the snRNA nucleic acid molecule according to any one of claims 1 to 46, the combination according to claim 47 or 48, the DNA molecule according to claim 49, the gene expression cassette according to any one of claims 50 to 54, the recombinant expression vector according to claims 55 to 59, the virus particle according to any one of claims 58 to 61, or the pharmaceutical composition according to claim 64 or 65.

71. A method for inducing splicing skipping of exon 13 of USH2A pre-mRNA, comprising administering the snRNA nucleic acid molecule according to any one of claims 1 to 46, the combination according to claim 47 or 48, the DNA molecule according to claim 49, the gene expression cassette according to any one of claims 50 to 54, the recombinant expression vector according to claims 55 to 59, the virus particle according to any one of claims 60 to 63, or the pharmaceutical composition according to claim 64 or 65.

72. A method for reducing the abnormal expression of an Usherin protein, comprising introducing into a host cell the snRNA nucleic acid molecule according to any one of claims 1 to 46, the combination according to claim 47 or 48, the DNA molecule according to claim 49, the gene expression cassette according to any one of claims 50 to 54, the recombinant expression vector according to claims 55 to 59, the virus particle according to any one of claims 60 to 63, or the pharmaceutical composition according to claim 64 or 65.

73. The method according to claim 72, wherein the host cell is selected from retinal tissue cells, inner ear cells, cells with differentiation potential into retinal tissue cells and/or inner ear cells, and cells capable of performing functions corresponding to retinal tissue cells and/or inner ear cells.

74. The method according to claim 73, wherein the retinal tissue cells are retinal photoreceptor cells, and the inner ear cells are inner ear hair cells.

75. The method according to claim 74, wherein the cells with differentiation potential are selected from induced pluripotent stem cells, embryonic stem cells, neural precursor cells, retinal progenitor cells, retinal precursor cells, and mesenchymal stromal cells.

76. A method for preparing the snRNA nucleic acid molecule according to any one of claims 1 to 46 or the combination according to claim 47 or 48, comprising the step of biosynthesis or chemical synthesis of the snRNA nucleic acid molecule according to any one of claims 1 to 44 or the combination according to claim 47 or 48.

77. Use of the snRNA nucleic acid molecule according to any one of claims 1 to 46, the combination according to claim 47 or 48, the DNA molecule according to claim 49, the gene expression cassette according to any one of claims 50 to 54, the recombinant expression vector according to claims 55 to 59, the virus particle according to any one of claims 60 to 63, or the pharmaceutical composition according to claim 64 or 65 in the manufacture of a medicament for treating a disease associated with a mutation in exon 13 of USH2A.

78. The use according to claim 77, wherein the mutation in exon 13 of USH2A is a pathogenic mutation or a non-pathogenic mutation.

79. The use according to claim 77, wherein the disease is selected from eye diseases and ear diseases.
